(19) Europäisches Patentamt
European Patent Office
Office européen des brevets

(11) EP 1 129 194 B1

(12) # EUROPEAN PATENT SPECIFICATION

(45) Date of publication and mention of the grant of the patent:
**28.09.2005 Bulletin 2005/39**

(21) Application number: **99956675.5**

(22) Date of filing: **27.10.1999**

(51) Int Cl.[7]: **C12N 15/25**, C07K 14/545, A61K 38/20, C12N 5/10, C12N 15/62 // A61P37/02

(86) International application number:
**PCT/US1999/025038**

(87) International publication number:
**WO 2000/024899 (04.05.2000 Gazette 2000/18)**

(54) **INTERLEUKIN-1 HOMOLOG ZIL1A4**

ZIL 1A4 EIN HOMOLOG DES INTERLEUKIN-1

HOMOLOGUES ZIL 1A4 D'INTERLEUKINE-1

(84) Designated Contracting States:
**AT BE CH CY DE DK ES FI FR GB GR IE IT LI LU MC NL PT SE**

(30) Priority: **27.10.1998 US 179614**

(43) Date of publication of application:
**05.09.2001 Bulletin 2001/36**

(73) Proprietor: **ZymoGenetics, Inc.
Seattle, WA 98102 (US)**

(72) Inventors:
- **WEST, Robert, R.
Seattle, WA 98117 (US)**
- **SHEPPARD, Paul, O.
Granite Falls, WA 98252 (US)**
- **GAO, Zeren
Redmond, WA 98053 (US)**

(74) Representative: **Dean, John Paul et al
Withers & Rogers LLP
Goldings House,
2 Hays Lane
London SE1 2HW (GB)**

(56) References cited:
**WO-A-99/06426**

- **"ou40f01.x1 Soares_NFL_T_GBC_S1 Homo sapiens cDNA clone IMAGE:1628761 3' " EMBL DATABASE ACCESSION NUMBER AI014548, 18 June 1998 (1998-06-18), XP002135000**
- **DINARELLO C A: "BIOLOGIC BASIS FOR INTERLEUKIN-1 IN DISEASE" BLOOD, vol. 87, no. 6, 15 March 1996 (1996-03-15), pages 2095-2147, XP000574933 ISSN: 0006-4971**
- **PRYOR KD. ET AL.: "High-level expression of soluble protein in Escherichia coli using a His6-tag and maltose-binding-protein double-affinity fusion system." PROTEIN EXPR PURIF 10(3):309-19, August 1997 (1997-08), XP000891558 cited in the application**
- **SMITH DE. ET AL.: "Four new members expand the interleukin-1 superfamily." J BIOL CHEM 2000 JAN 14;275(2):1169-75, XP002135001**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

BACKGROUND OF THE INVENTION

In multicellular organisms, cell growth, differentiation, migration, and metabolism are controlled by a variety of polypeptide factors. These factors regulate both normal development and pathogenesis.

The interleukins are a family of cytokines that mediate immunological responses, including inflammation. Interleukin-1 (IL-1) is a family of at least three known members with recognizable sequence homology and common receptor binding activities. IL-1α and IL-1β are pro-inflammatory cytokines, while the third family member, IL-1 receptor antagonist (IL-1ra) is an antagonist of IL-1α and IL-1β activities. 1L-1ra is unusual in that it is the only known, naturally occuring cytokine receptor antagonist with no apparent agonist function. The ability of IL-1ra to bind, but not activate, the IL-1 receptor suggests that IL-1ra is a negative regulator of inflammation (Dripps et al., *J. Biol. Chem.* 266: 10331-10336, 1991; Granowitz et al., *Blood* 79:2356-2363, 1992).

The interleukins mediate a variety of inflammatory pathologies. At low concentrations Il-1α and IL-1β act locally on mononuclear phagocytes and the vascular endothelium to induce further IL-1 and IL-6 synthesis. IL-1 does not act directly on leukocytes and neutrophils, but causes the mononuclear phagocytes and endothelial cells to activate leukocytes. When secreted in large quantities into the bloodstream, the IL-1 has endocrine effects, including fever, synthesis of acute phase plasma proteins, and cachexia.

Early reports described naturally occurring inhibitors of IL-1 activity in many sources: urine from febrile patients (Seckinger et al., *J. Immunol*. 139:1546-1549, 1987; Mazzei et al., *Eur. J. Immunol*. 20:683-689, 1990), monocytic leukemia patients (Seckinger et al., *ibid.*)*,* or juvenile chronic arthritis patients (Prieur et al., *Lancet* 2:1240-1242, 1987); synovial fluid of rheumatoid arthritis patients (Lotz et al., *J. Clin. Invest*. 78:713-721, 1986); and viral-infected monocytes or B-cells (Scala et al., *J. Exp. Med.* 159:1637-1652, 1984). These inhibitory bioactivities appear to be heterogeneous with a range of molecular mass from 18 to 67 kDa, but they remain largely uncharacterized. The first demonstration of a true IL-1 receptor antagonist came from the purification and cloning of recombinant IL-1ra from human peripheral blood mononuclear cells (PMNC) cultured on IgG-coated plates and from the human monocytic cell line U937 activated with phorbol ester (PMA) (Carter et al., *Nature* 344:633-638, 1990; Hannum et al., *Nature* 343:336-340, 1990; Eisenberg et al., *Nature* 343:341-346, 1990).

Serum from healthy people has a low level of circulating IL-1ra activity (200-400 pg/ml). Serum IL-1ra levels are dramatically increased in patients with acute or chronic inflammatory disease, certain cancers, infectious diseases, and septic shock (Fisher et al., *Blood* 79:2196-2200, 1992), major surgery for Hirshsprung's disease (O Nuallain et al., *Clin. Exp. Immunol*. 93:218-222, 1993), liver disease (Sekiyama et al., *Clin. Exp. Immunol*. 98:71-77, 1994), Hodgkin's disease (Gruss et al., *Lancet* 340:968, 1992), and colitis (Hyams et al., *Dig. Dis. Sci.* 39:1893-1899, 1994). Other acute and chronic disorders also induce local production of IL-1ra, for example, rheumatoid arthritis (Firestein et al., *J. Immunol.* 149:1054-1062 1992), Lyme arthritis (Miller et al., *Lancet* 341:146-148, 1993), and nonperforating Crohn's disease (Gilberts et al., *Proc. Natl. Acad. Sci. USA* 91: 12721-12724, 1994). Induction of endotoxemia in human volunteers induces 100-fold excesses of IL-1ra over IL-1β in serum, however, this level is still apparently insufficient to abolish IL-1 activity *in vivo* (Granowitz et al., *Lancet* 338:1423-1424, 1991). Numerous experiments have shown the potential for systemic, intravenous injection of IL-1ra to attenuate the effects of administered IL-1 or LPS in animal models of endotoxaemia or synovitis (Dinarello and Thompson, *Immunol. Today* 12:404-410, 1991). When antibodies to IL-1ra were administered iv in a rabbit formalin-induced colitis model there was significant exacerbation of intestinal inflammation and mortality (Ferretti et al., *J. Clin. Invest.* 94:449-453, 1994).

IL-1ra has been investigated for use in treating several chronic inflammatory disorders including: rheumatoid arthritis (Henderson et al., *Cytokine* 3:246-249, 1991), chronic myelogenous leukemia (CML) (Schiro et al., *Blood* 83: 460-465, 1994), and inflammatory bowel disease (IBD) (Cominelli et al., *Gastroenterology* 103:65-71, 1992). Some evidence also suggests that IL-1ra may be useful in the treatment of psoriasis. Normal skin expresses IL-1ra mainly in the differentiated stratum granulosum of the epidermis, whereas psoriatic skin expresses IL-1ra in basal midbasal layers (Hammerberg et al, *J. Clin. Invest.* 90:571-583, 1992). Changes in the IL-1α:IL1-ra ratio in different strata of the epidermis may affect keratinocyte proliferation and differentiation. Chronic inflammatory bowel disease may also involve an altered IL-1:Il-1ra ratio since it is markedly increased in Crohn's disease and ulcerative colitis (Cominelli et al., *Cytokine* 6:A171, 1994). Experimental evidence also suggests that IL-1ra may be useful in chronic and acute cerebral neuropathologies (Relton et al., *Exp. Neurol.* 138:206-213, 1996; Loddick et al., *Biochem. Biophys. Res. Comm.* 234:211-215, 1997), insulin dependent diabetes mellitus (Madrup-Poulsen et al., *Cytokine* 5:185-191, 1993), glomerulonephritis (Lan et al., *Kidney Int*. 47:1303-1309, 1995), and pancreatitis (Norman et al., *Ann. Surg.* 221:625, 1995).

Increased IL-1 production has been reported in patients with various viral, bacterial, fungal, and parasitic infections; intravascular coagulation; high-dose IL-2 therapy; solid tumors; leukemias Alzheimer;s disease; HIV-1 infection; autoimmune disorders; trauma (surgery); hemodialysis; ischemic diseases (myocardial infarction); noninfectious hepatitis; asthma; UV radiation; closed head injury; pancreatitis; peridontitis; graft-versus-host disease; transplant rejection; and in healthy subjects after strenuous exercise (Dinarello, *Blood* 87:2095-2147, 1996).

Recombinant IL-1ra has been shown to be well tolerated in clinical trials in humans (Campion et al., *Arthritis and Rhematism* 39: 1092-1101, 1996), and to be potentially efficacious in the treatment of septic shock (Fisher et al., *JAMA* 271:1836-1843, 1994), rheumatoid arthritis (Campion et al., *Arthritis & Rheumatism* 39:1092-1101, 1996), and graft vs. host disease (GVHD) (Antin et al., *Blood* 84:1342-1348, 1994). However, high serum concentrations of the molecule are often required because of receptor binding affinity, plasma half-life, and tissue permeability. There thus remains a need in the art for additional molecules that modulate inflammatory and other immunological processes.

## SUMMARY OF THE INVENTION

The present invention provides a family of novel interleukin-1 (IL-1) homologs, as well as materials and methods for making the IL-1 homologs, compositions comprising them, and methods for using them. Within one of its aspects, the invention thus provides an isolated protein comprising a sequence of amino acid residues selected from the group consisting of residues 60 through 218 of SEQ ID NO:7 and residues 1 through 157 of SEQ ID NO:10. Within one embodiment of the invention the amino acid residue corresponding to residue number 200 of SEQ ID NO:2 is Lys. Within another embodiment of the invention the amino acid residue corresponding to residue number 200 of SEQ ID NO:2 is Asp. Within a further embodiment of the invention the amino acid residue corresponding to residue number 200 of SEQ ID NO:2 is Glu. Within another embodiment of the invention the protein comprises residues 60 through 218 of SEQ ID NO:11. Exemplary proteins in this regard include those comprising residues 60 through 218 of SEQ ID NO:14 or SEQ ID NO:2. Within additional embodiments of the invention the protein comprises residues 1 through 218 of SEQ ID NO:11 or residues 1 through 157 of SEQ ID NO:12. Exemplary proteins in this regard include those comprising residues 1 through 218 of SEQ ID NO:14, residues 1 through 218 of SEQ ID NO:2, residues 1 through 157 of SEQ ID NO:15, or residues 1 through 157 of SEQ ID NO:9. Within other embodiments the protein is from 157 to 1500 amino acid residues in length. The proteins may further comprise one or more affinity tags.

Within a second aspect of the invention there is provided an isolated polynucleotide encoding a protein comprising a sequence of amino acid residues selected from the group consisting of residues 60 through 218 of SEQ ID NO:7 and residues 1 through 157 of SEQ ID NO:10. Within one embodiment the isolated polynucleotide is DNA. Within other embodiments the protein encoded by the polynucleotide comprises a sequence of amino acid residues selected from the group consisting of residues 60 through 218 of SEQ ID NO:11 and residues 1 through 157 of SEQ ID NO:12. Exemplary proteins include those comprising residues 1 through 218 of SEQ ID NO:14, residues 1 through 218 of SEQ ID NO:2, residues 1 through 157 of SEQ ID NO:15, or residues 1 through 157 of SEQ ID NO:9.

Within a third aspect of the invention there is provided an expression vector comprising the following operably linked elements: a transcription promoter; a DNA segment encoding a protein as disclosed above; and a transcription terminator. Within one embodiment the vector further comprises a secretory signal sequence operably linked to said DNA segment.

Within a fourth aspect of the invention there is provided a cultured cell comprising an expression vector according as disclosed above. Also provided is a method of making a protein comprising culturing the cell under conditions wherein the DNA segment is expressed, and recovering the protein encoded by said DNA segment.

Within a fifth aspect of the invention there is provided a method of modulating an immune response in an animal comprising administering to said animal a composition comprising a protein as disclosed above in combination with a pharmaceutically acceptable vehicle.

These and other aspects of the invention will become evident upon reference to the following detailed description of the invention and the attached drawings.

## BRIEF DESCRIPTION OF THE DRAWINGS

Fig. 1 is a Hopp/Woods hydrophilicity profile of the amino acid sequence shown in SEQ ID NO:2. The profile is based on a sliding six-residue window. Buried G, S, and T residues and exposed H, Y, and W residues were ignored. These residues are indicated in the figure by lower case letters.

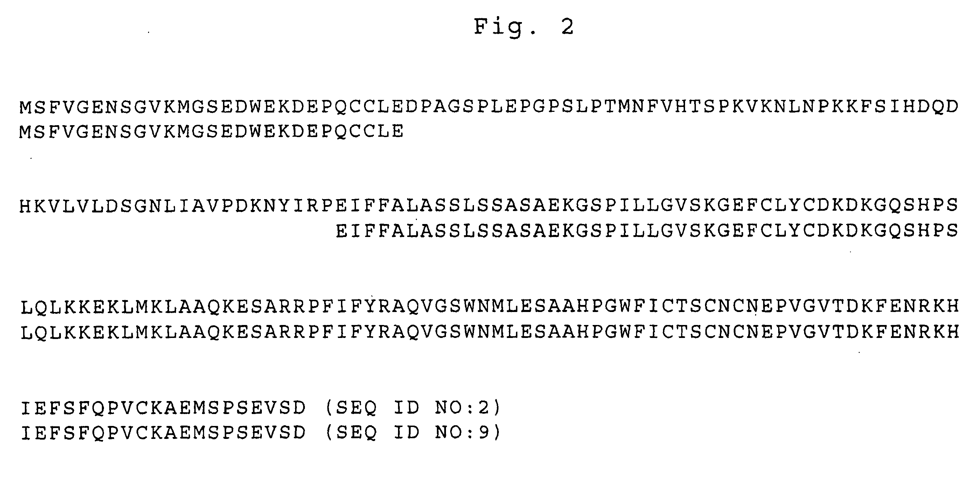

Fig. 2 is an alignment of two alternatively spliced forms of human zil1a4 (SEQ ID NO:2 and SEQ ID NO:9). Amino acid residues are represented by the standard one-letter codes.

## DETAILED DESCRIPTION OF THE INVENTION

The term "affinity tag" is used herein to denote a polypeptide segment that can be attached to a second polypeptide to provide for purification of the second polypeptide or provide sites for attachment of the second polypeptide to a substrate. In principal, any peptide or protein for which an antibody or other specific binding agent is available can be used as an affinity tag. Affinity tags include a poly-histidine tract, protein A (Nilsson et al., *EMBO J.* 4:1075, 1985; Nilsson et al., *Methods Enzymol.* 198:3, 1991), glutathione S transferase (Smith and Johnson, *Gene* 67:31, 1988), Glu-Glu affinity tag (Grussenmeyer et al., *Proc. Natl. Acad. Sci. USA* 82:7952-4, 1985) (SEQ ID NO:42), substance P, Flag™ peptide (Hopp et al., *Biotechnology* 6:1204-1210, 1988), streptavidin binding peptide, maltose binding protein (Guan et al., *Gene* 67:21-30, 1987), cellulose binding protein, thioredoxin, ubiquitin, T7 polymerase, or other antigenic epitope or binding domain. See, in general, Ford et al., *Protein Expression and Purification* 2: 95-107, 1991. DNAs encoding affinity tags and other reagents are available from commercial suppliers (e.g., Pharmacia Biotech, Piscataway, NJ; New England Biolabs, Beverly, MA; Eastman Kodak, New Haven, CT).

The term "allelic variant" is used herein to denote any of two or more alternative forms of a gene occupying the same chromosomal locus. Allelic variation arises naturally through mutation, and may result in phenotypic polymorphism within populations. Gene mutations can be silent (no change in the encoded polypeptide) or may encode polypeptides having altered amino acid sequences. The term allelic variant is also used herein to denote a protein encoded by an allelic variant of a gene.

The term "complement of a polynucleotide molecule" denotes a polynucleotide molecule having a complementary base sequence and reverse orientation as compared to a reference sequence. For example, the sequence 5' ATGCACGGG 3' is complementary to 5' CCCGTGCAT 3'.

The term "corresponding to", when applied to positions of amino acid residues in sequences, means corresponding positions in a plurality of sequences when the sequences are optimally aligned.

The term "degenerate nucleotide sequence" denotes a sequence of nucleotides that includes one or more degenerate codons (as compared to a reference polynucleotide molecule that encodes a polypeptide). Degenerate codons contain different triplets of nucleotides, but encode the same amino acid residue (i.e., GAU and GAC triplets each encode Asp).

The term "expression vector" is used to denote a DNA molecule, linear or circular, that comprises a segment encoding a polypeptide of interest operably linked to additional segments that provide for its transcription. Such additional segments include promoter and terminator sequences, and may also include one or more origins of replication, one or more selectable markers, an enhancer, a polyadenylation signal, etc. Expression vectors are generally derived from plasmid or viral DNA, or may contain elements of both.

The term "isolated", when applied to a polynucleotide, denotes that the polynucleotide has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art (see for example, Dynan and Tijan, *Nature* 316:774-78, 1985).

An "isolated" polypeptide or protein is a polypeptide or protein that is found in a condition other than its native environment, such as apart from blood and animal tissue. In a preferred form, the isolated polypeptide is substantially free of other polypeptides, particularly other polypeptides of animal origin. It is preferred to provide the polypeptides in a highly purified form, i.e. greater than 95% pure, more preferably greater than 99% pure. When used in this context, the term "isolated" does not exclude the presence of the same polypeptide in alternative physical forms, such as dimers or alternatively glycosylated or derivatized forms.

A "motif" is a series of amino acid positions in a protein sequence for which certain amino acid residues are required. A motif defines the set of possible residues at each such position.

The term "operably linked", when referring to DNA segments, indicates that the segments are arranged so that they function in concert for their intended purposes, e.g., transcription initiates in the promoter and proceeds through the coding segment to the terminator.

The term "ortholog" denotes a polypeptide or protein obtained from one species that is the functional counterpart of a polypeptide or protein from a different species. Sequence differences among orthologs are the result of speciation.

A "polynucleotide" is a single- or double-stranded polymer of deoxyribonucleotide or ribonucleotide bases read from the 5' to the 3' end. Polynucleotides include RNA and DNA, and may be isolated from natural sources, synthesized *in vitro*, or prepared from a combination of natural and synthetic molecules. Sizes of polynucleotides are expressed as base pairs (abbreviated "bp"), nucleotides ("nt"), or kilobases ("kb"). Where the context allows, the latter two terms may describe polynucleotides that are single-stranded or double-stranded. When the term is applied to double-stranded molecules it is used to denote overall length and will be understood to be equivalent to the term "base pairs". It will be recognized by those skilled in the art that the two strands of a double-stranded polynucleotide may differ slightly in length and that the ends thereof may be staggered as a result of enzymatic cleavage; thus all nucleotides within a double-stranded polynucleotide molecule may not be paired. Such unpaired ends will in general not exceed 20 nt in length.

A "polypeptide" is a polymer of amino acid residues joined by peptide bonds, whether produced naturally or synthetically. Polypeptides of less than about 10 amino acid residues, are commonly referred to as "peptides".

The term "promoter" is used herein for its art-recognized meaning to denote a portion of a gene containing DNA sequences that provide for the binding of RNA polymerase and initiation of transcription. Promoter sequences are commonly, but not always, found in the 5' non-coding regions of genes.

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

The term "receptor" denotes a cell-associated protein that binds to a bioactive molecule (i.e., a ligand) and mediates the effect of the ligand on the cell.

The term "secretory signal sequence" denotes a DNA sequence that encodes a polypeptide (a "secretory peptide") that, as a component of a larger polypeptide, directs the larger polypeptide through a secretory pathway of a cell in which it is synthesized. The larger polypeptide is commonly cleaved to remove the secretory peptide during transit through the secretory pathway.

A "segment" is a portion of a larger molecule (e.g., polynucleotide or polypeptide) having specified attributes. For example, a DNA segment encoding a specified polypeptide is a portion of a longer DNA molecule, such as a plasmid or plasmid fragment, that, when read from the 5' to the 3' direction, encodes the sequence of amino acids of the specified polypeptide.

The term "splice variant" is used herein to denote alternative forms of RNA transcribed from a gene. Splice variation arises naturally through use of alternative splicing sites within a transcribed RNA molecule, or less commonly between separately transcribed RNA molecules, and may result in several mRNAs transcribed from the same gene. Splice variants may encode polypeptides having altered amino acid sequence. The term splice variant is also used herein to denote a protein encoded by a splice variant of an mRNA transcribed from a gene.

Molecular weights and lengths of polymers determined by imprecise analytical methods (e.g., gel electrophoresis) will be understood to be approximate values. When such a value is expressed as "about" X or "approximately" X, the stated value of X will be understood to be accurate to $\pm 10\%$.

The present invention provides a group of novel proteins, designated "zil1a4", that are members of the IL-1 family. Analysis of a representative human zil1a4 sequence (SEQ ID NO:1 and NO:2) indicates that this protein, like other members of the family, contains a core structure of 12 β-strands wound into a β-barrel, with the β-strands separated from each other by loops. The loops between these β-strands are highly variable among the family members and are believed to be involved in receptor binding. These loops, which each contain at least three amino acid residues and may contain up to 17 residues, do not form β-strands or helices, but may (and often do) contain β-turns. Referring to SEQ ID NO:2, the twelve β-strands are formed by residues 60-64, 68-72, 77-79, 90-96, 108-113, 118-123, 132-138, 154-160, 165-169, 175-179, 187-189, and 201-204. These strands are characterized by a high content of hydrophobic amino acid residues (Leu, Val, Phe, and Ile). The loops include residues 65-67, 73-76, 80-89, 97-107, 114-117, 124-131, 139-153, 161-164, 170-174, 180-186, and 190-200.

In addition to the β-strands and loops, the zil1a4 proteins are characterized by the presence of conserved motifs at positions corresponding to (1) residues 167-171 of SEQ ID NO:2, (2) residues 173-177 of SEQ ID NO:2, (3) residues 186-190 of SEQ ID NO:2, and (4) residues 199-204 of SEQ ID NO:2. These motifs are shown in Table 1 using the standard single-letter codes for amino acid residues. Square brackets indicate alternative residues at a given position within the motif.

Table 1

| Motif | Sequence | SEQ ID NO: |
|---|---|---|
| 1 | [LF] [ET] S [AV] [AQ] | 3 |
| 2 | P [GN] [LW] [FY] [IL] | 4 |
| 3 | [PW] [VL] [SCFRIG] [LV] [TAG] | 5 |
| 4 | [TI] [DKE] F [SYTQ] [FMI] [QNIL] | 6 |

The present invention thus provides a family of zil1a4 proteins as shown in SEQ ID NO:7.

The higher-order structure of the IL-1 family of proteins can also be envisioned as three 4-stranded covalent monomers assembled into a 12-stranded structure (a "trefoil"). When these monomers are superimposed on each other, residues 90 (Ile), 155 (Ile), and 200 (Glu) of SEQ ID NO:2 occupy like positions in their respective monomers.

A second, alternatively spliced form of human zil1a4 is shown in SEQ ID NO:8 and SEQ ID NO:9. Comparison of this shorter form with the protein of SEQ ID NO:2 and analysis of the corresponding genomic sequence indicates that the DNA of SEQ ID NO:8 is lacking two exons as compared to SEQ ID NO:1. The amino acid sequence shown in SEQ ID NO:9 can be varied as shown in Table 1. The invention thus provides a second family of zil1a4 proteins as shown in SEQ ID NO:10.

The proteins of the present invention modulate immunological responses. As used herein, "immunological responses" includes one or more of cell proliferation, cell differentiation, cell maturation, and cell activation. Modulation of immunological responses includes modulation of inflammation. These indicators of immunological responses are measured by methods known in the art and disclosed in more detail herein.

Proteins of the present invention are expected to have pro-inflammatory (agonist) or anti-inflammatory (antagonist) activity, depending on the particular amino acid sequence. In general, zil1a4 proteins having a Lys residue corresponding to position 200 of SEQ ID NO:2 (position 139 of SEQ ID NO:9) will have anti-inflammatory activity, while those having a Glu residue at this position will have pro-inflammatory activity. Pro-inflammatory activity may be attenuated by substituting an Asp for Glu at this position. Sequence variations at positions corresponding to residues 90 and 155 of SEQ ID NO:2 (residues 29 and 94 of SEQ ID NO:9, respectively) may also influence biological activity. Within certain embodiments the present invention also includes zil1a4 proteins having Lys, Glu, or Ile at a position corresponding to residue 90 of SEQ ID NO:2 and Ala, Ile, or Thr at a position corresponding to residue 155 of SEQ ID NO:2. Sequences of zil1a4 proteins having substitutions at amino acid residues corresponding to positions 90, 155, and 200 of SEQ ID NO:2 are shown in SEQ ID NO:11 and SEQ ID NO:12. While not wishing to be bound by theory, it is believed that zil1a4 proteins act through IL-1 receptors or IL-1 homolog receptors. These receptors are characterized by the presence of C-2 type immunoglobulin domains and include IL-1 receptor type I (Sims et al., *Proc. Natl. Acad.* Sci. *USA* 86:8946-8950, 1989), IL-1 receptor type II (McMahan et al., *EMBO J.* 10:2821-2832, 1991), ST2 (Tominaga et al., *Biochim. Biophys. Acta* 1171:215-218, 1992), opiod binding protein/cell adhesion molecule (Shark and Lee, *Gene* 155:213-217, 1995), and basic FGF receptor (Isacchi et al., *Nuc. Acids Res.* 18:1906, 1990; Dionne et al., *EMBO J.* 9:2685-2692, 1990). Pro- and anti-inflammatory activities can be assayed using standard assays of IL-1 activity known in the art.

While not wishing to be bound by theory, the Met residues at positions 1, 12, and 43 of SEQ ID NO:2 may represent alternative translation start sites. Residues 54 and 60 follow potential proteolytic processing sites. The present invention thus provides polypeptides that comprise amino acid residues 60-218 of SEQ ID NO:7, preferably residues 60-218 of SEQ ID NO:11, more preferably residues 60-218 of SEQ ID NO:2 or SEQ ID NO:14. The invention also provides polypeptides that comprise amino acid residues 54-218 of SEQ ID NO:7, preferably residues 54-218 of SEQ ID NO:11, more preferably residues 54-218 of SEQ ID NO:2 or SEQ ID NO:14. The invention also provides polypeptides that comprise amino acid residues 43-218 of SEQ ID NO:7, preferably residues 43-218 of SEQ ID NO:11, more preferably residues 43-218 of SEQ ID NO:2 or SEQ ID NO:14; and polypeptides that comprise amino acid residues 12-218 of SEQ ID NO:7, preferably residues 12-218 of SEQ ID NO:11, more preferably residues 12-218 of SEQ ID NO:2 or SEQ ID NO:14. These proteins comprise motifs 1-4 disclosed above at positions corresponding to residues 167-171, 173-177, 186-190, and 199-204, respectively, of SEQ ID NO:2. In addition, the proteins comprise β-strands at positions corresponding to residues 60-64, 68-72, 77-79, 90-96, 108-113, 118-123, 132-138, 154-160, 165-169, 175-179, 187-189, and 201-204 of SEQ ID NO:2, wherein these β-strands are separated from each other by the aforementioned loops.

Those skilled in the art will recognize that minor modifications can be made in the proteins of the present invention without altering biological function. For example, by varying the lengths of the various regions of the molecule, in particular the loops and other sequences not defined by β-strands or motifs, the length of the zil1a4 protein can be varied. Small extensions can also be made in the β-strands. It is preferred to limit the extent of amino acid substitutions, insertions, and deletions so that the resulting proteins are at least 95% identical to residues 60-218 of SEQ ID NO:7 or residues 1 to 157 of SEQ ID NO:10.

Percent sequence identity is determined by conventional methods. See, for example, Altschul et al., *Bull. Math. Bio.* 48:603-616, 1986, and Henikoff and Henikoff, *Proc. Natl. Acad. Sci.* USA 89:10915-10919, 1992. Briefly, two amino acid sequences are aligned to optimize the alignment scores using a gap opening penalty of 10, a gap extension penalty of 1, and the "BLOSUM62" scoring matrix of Henikoff and Henikoff (*ibid*.) as shown in Table 2 (amino acids are indicated by the standard one-letter codes). The percent identity is then calculated as:

$$\frac{\text{Total number of identical matches}}{\text{[length of the longer sequence plus the number of gaps introduced into the longer sequence in order to align the two sequences]}} \times 100$$

<u>Table 2</u>

| | A | R | N | D | C | Q | E | G | H | I | L | K | M | F | P | S | T | W | Y | V |
|---|---|---|---|---|---|---|---|---|---|---|---|---|---|---|---|---|---|---|---|---|
| A | 4 | | | | | | | | | | | | | | | | | | | |
| R | -1 | 5 | | | | | | | | | | | | | | | | | | |
| N | -2 | 0 | 6 | | | | | | | | | | | | | | | | | |
| D | -2 | -2 | 1 | 6 | | | | | | | | | | | | | | | | |
| C | 0 | -3 | -3 | -3 | 9 | | | | | | | | | | | | | | | |
| Q | -1 | 1 | 0 | 0 | -3 | 5 | | | | | | | | | | | | | | |
| E | -1 | 0 | 0 | 2 | -4 | 2 | 5 | | | | | | | | | | | | | |
| G | 0 | -2 | 0 | -1 | -3 | -2 | -2 | 6 | | | | | | | | | | | | |
| H | -2 | 0 | 1 | -1 | -3 | 0 | 0 | -2 | 8 | | | | | | | | | | | |
| I | -1 | -3 | -3 | -3 | -1 | -3 | -3 | -4 | -3 | 4 | | | | | | | | | | |
| L | -1 | -2 | -3 | -4 | -1 | -2 | -3 | -4 | -3 | 2 | 4 | | | | | | | | | |
| K | -1 | 2 | 0 | -1 | -3 | 1 | 1 | -2 | -1 | -3 | -2 | 5 | | | | | | | | |
| M | -1 | -1 | -2 | -3 | -1 | 0 | -2 | -3 | -2 | 1 | 2 | -1 | 5 | | | | | | | |
| F | -2 | -3 | -3 | -3 | -2 | -3 | -3 | -3 | -1 | 0 | 0 | -3 | 0 | 6 | | | | | | |
| P | -1 | -2 | -2 | -1 | -3 | -1 | -1 | -2 | -2 | -3 | -3 | -1 | -2 | -4 | 7 | | | | | |
| S | 1 | -1 | 1 | 0 | -1 | 0 | 0 | 0 | -1 | -2 | -2 | 0 | -1 | -2 | -1 | 4 | | | | |
| T | 0 | -1 | 0 | -1 | -1 | -1 | -1 | -2 | -2 | -1 | -1 | -1 | -1 | -2 | -1 | 1 | 5 | | | |
| W | -3 | -3 | -4 | -4 | -2 | -2 | -3 | -2 | -2 | -3 | -2 | -3 | -1 | 1 | -4 | -3 | -2 | 1 | 1 | |
| Y | -2 | -2 | -2 | -3 | -2 | -1 | -2 | -3 | 2 | -1 | -1 | -2 | -1 | 3 | -3 | -2 | -2 | 2 | 7 | |
| V | 0 | -3 | -3 | -3 | -1 | -2 | -2 | -3 | -3 | 3 | 1 | -2 | 1 | -1 | -2 | -2 | 0 | -3 | -1 | 4 |

The level of identity between amino acid sequences can be determined using the "FASTA" similarity search algorithm disclosed by Pearson and Lipman (*Proc. Natl. Acad. Sci. USA* 85:2444, 1988) and by Pearson (*Meth. Enzymol.* 183:63, 1990). Briefly, FASTA first characterizes sequence similarity by identifying regions shared by the query sequence (e.g., SEQ ID NO:2) and a test sequence that have either the highest density of identities (if the ktup variable is 1) or pairs of identities (if ktup=2), without considering conservative amino acid substitutions, insertions, or deletions. The ten regions with the highest density of identities are then rescored by comparing the similarity of all paired amino acids using an amino acid substitution matrix, and the ends of the regions are "trimmed" to include only those residues that contribute to the highest score. If there are several regions with scores greater than the "cutoff" value (calculated by a predetermined formula based upon the length of the sequence and the ktup value), then the trimmed initial regions are examined to determine whether the regions can be joined to form an approximate alignment with gaps. Finally, the highest scoring regions of the two amino acid sequences are aligned using a modification of the Needleman-Wunsch-Sellers algorithm (Needleman and Wunsch, *J. Mol. Biol.* 48:444, 1970; Sellers, *SIAM J. Appl. Math.* 26:787, 1974), which allows for amino acid insertions and deletions. Preferred parameters for FASTA analysis are: ktup=1, gap opening penalty=10, gap extension penalty=1, and substitution matrix=BLOSUM62. These parameters can be introduced into a FASTA program by modifying the scoring matrix file ("SMATRIX"), as explained in Appendix 2 of Pearson, 1990 (*ibid.*).

FASTA can also be used to determine the sequence identity of nucleic acid molecules using a ratio as disclosed above. For nucleotide sequence comparisons, the ktup value can range between one to six, preferably from three to six, most preferably three, with other parameters set as default.

It is preferred to substitute an amino acid residue with a residue of like character (a "conservative substitution"). The BLOSUM62 matrix (Table 2) is an amino acid substitution matrix derived from about 2,000 local multiple alignments of protein sequence segments, representing highly conserved regions of more than 500 groups of related proteins (Henikoff and Henikoff, *ibid.*). Thus, the BLOSUM62 substitution frequencies can be used to define conservative amino acid substitutions that may be introduced into the amino acid sequences of the present invention. As used herein, the term "conservative amino acid substitution" refers to a substitution represented by a BLOSUM62 value of greater than -1. For example, an amino acid substitution is conservative if the substitution is characterized by a BLOSUM62 value of 0, 1, 2, or 3. Preferred conservative amino acid substitutions are characterized by a BLOSUM62 value of at least one 1 (e.g., 1, 2 or 3), while more preferred conservative amino acid substitutions are characterized by a BLOSUM62 value of at least 2 (e.g., 2 or 3).

The proteins of the present invention can also comprise non-naturally occurring amino acid residues. Non-naturally occurring amino acids include, without limitation, *trans*-3-methylproline, 2,4-methanoproline, *cis*-4-hydroxyproline, *trans*-4-hydroxyproline, *N*-methylglycine, *allo*-threonine, methylthreonine, hydroxyethylcysteine, hydroxyethylhomocysteine, nitroglutarnine, homoglutamine, pipecolic acid, thiazolidine carboxylic acid, dehydroproline, 3- and 4-methylproline, 3,3-dimethylproline, *tert*-leucine, norvaline, 2-azaphenylalanine, 3-azaphenylalanine, 4-azaphenylalanine, and 4-fluorophenylalanine. Several methods are known in the art for incorporating non-naturally occurring amino acid residues into proteins. For example, an *in vitro* system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations is carried out in a cell-free system comprising an *E. coli* S30 extract and commercially available enzymes and other reagents. Proteins are purified by chromatography. See, for example, Robertson et al., *J. Am. Chem. Soc.* 113:2722, 1991; Ellman et al., *Methods Enzymol.* 202:301, 1991; Chung et al., *Science* 259:806-9, 1993; and Chung et al., *Proc. Natl. Acad. Sci. USA* 90:10145-9, 1993). In a second method, translation is carried out in *Xenopus* oocytes by microinjection of mutated mRNA and chemically aminoacylated suppressor tRNAs (Turcatti et al., *J. Biol. Chem.* 271:19991-8, 1996). Within a third method, *E. coli* cells are cultured in the absence of a natural amino acid that is to be replaced (e.g., phenylalanine) and in the presence of the desired non-naturally occurring amino acid(s) (e.g., 2-azaphenylalanine, 3-azaphenylalanine, 4-azaphenylalanine, or 4-fluorophenylalanine). The non-naturally occurring amino acid is incorporated into the protein in place of its natural counterpart. See, Koide et al., *Biochem.* 33:7470-6, 1994. Naturally occurring amino acid residues can be converted to non-naturally occurring species by *in vitro* chemical modification. Chemical modification can be combined with site-directed mutagenesis to further expand the range of substitutions (Wynn and Richards, *Protein Sci.* 2:395-403, 1993).

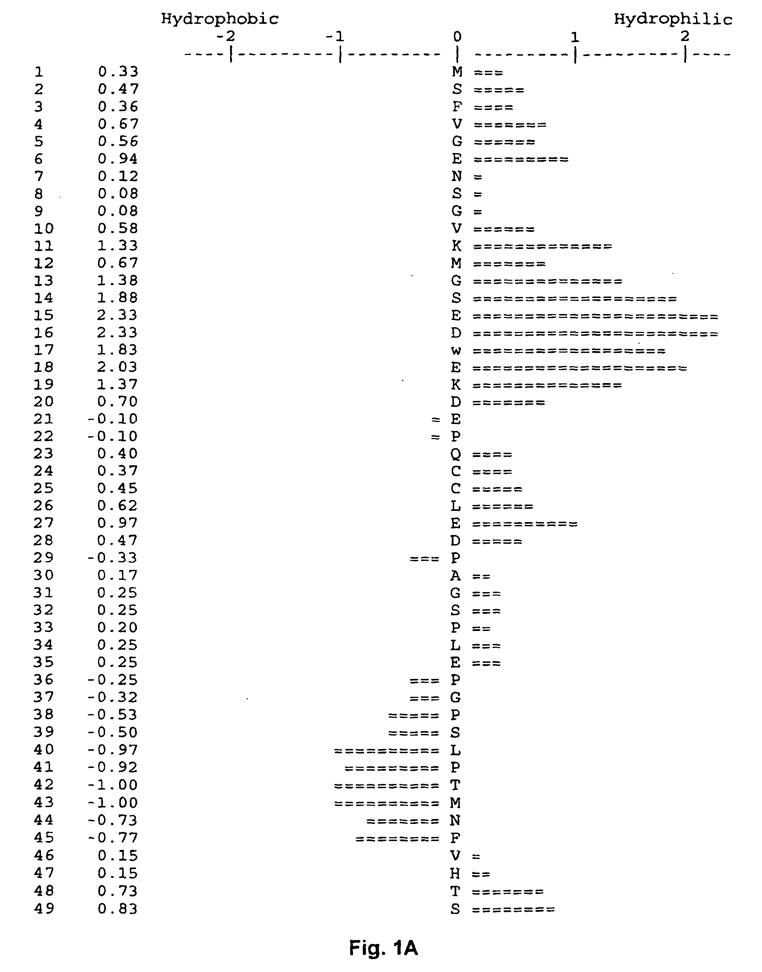

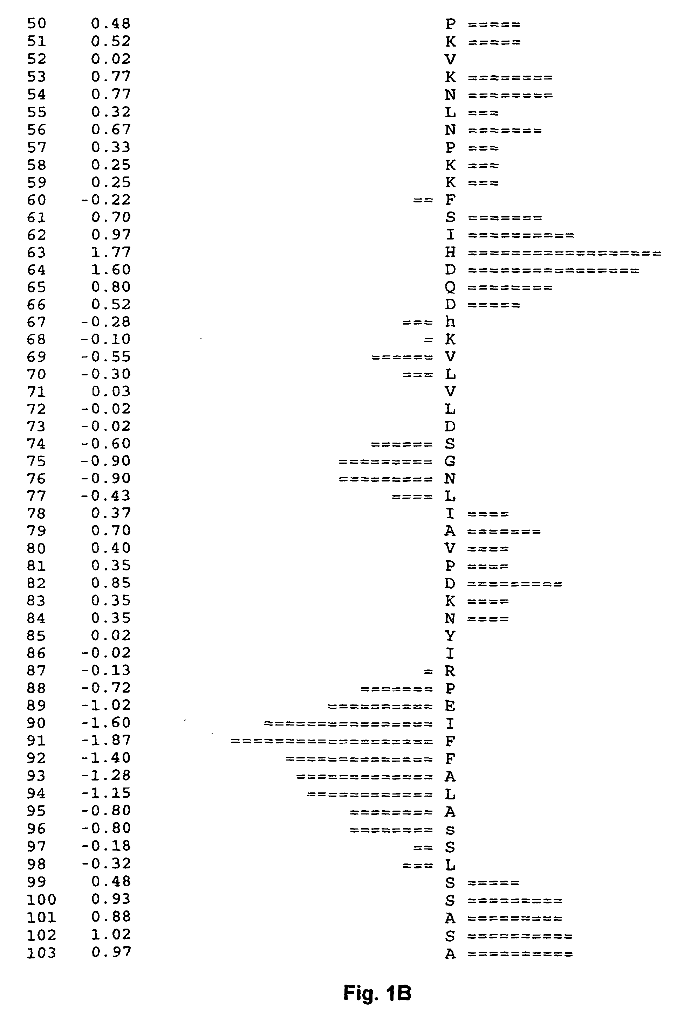

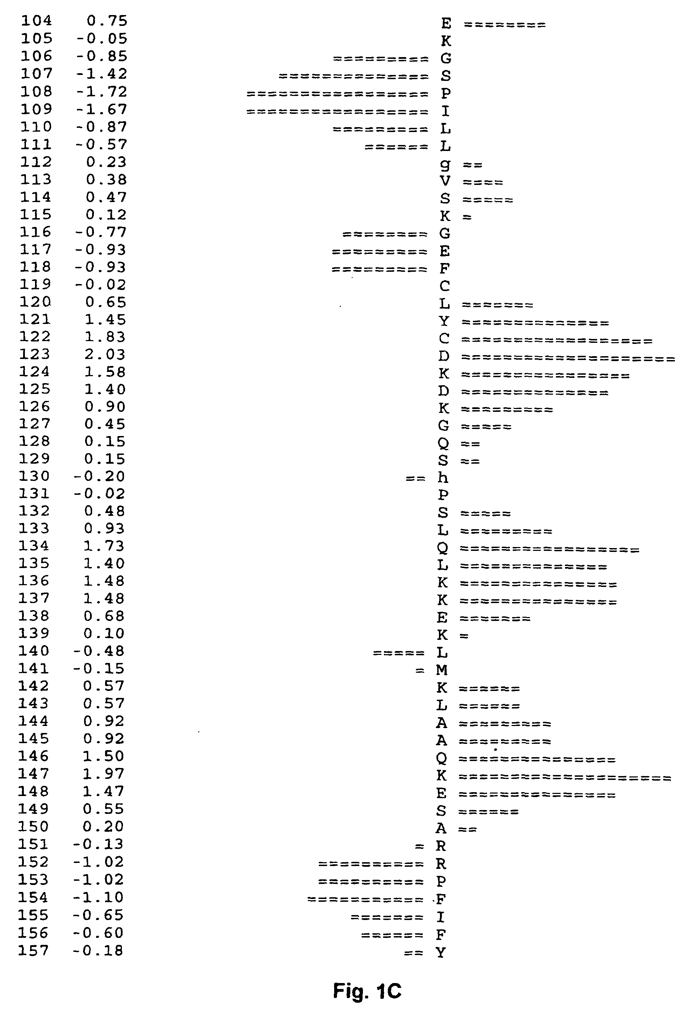

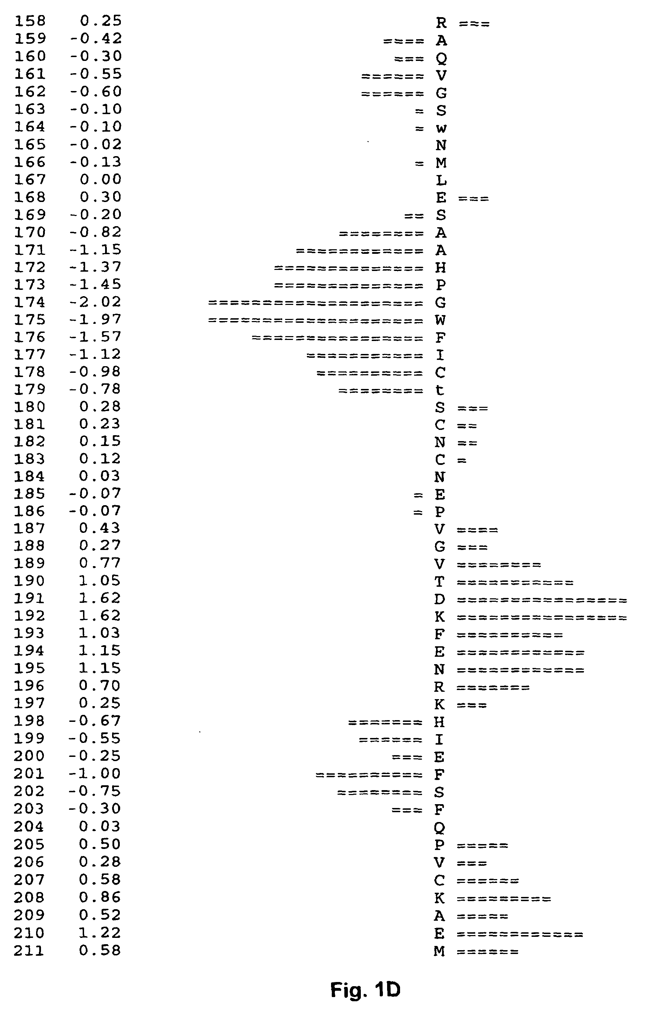

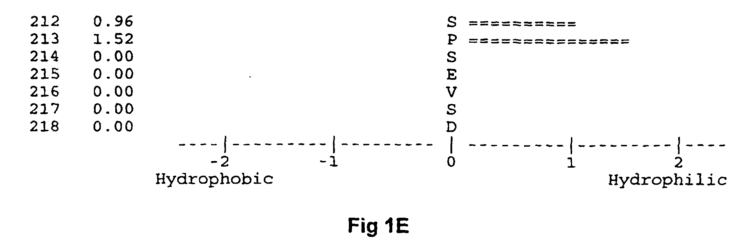

Within the present invention amino acid sequence changes can be made in the zil1a4 sequence shown in SEQ ID NO:2 to obtain other zil1a4 proteins. These changes are made so as to minimize disruption of higher order structure essential to biological activity. In particular, the arrangement of β-strands and loops will not be disrupted, thus it is preferred to make conservative amino acid substitutions within the β-strands, particularly when replacing hydrophobic residues. Those skilled in the art will recognize that hydrophobic and aromatic residues can sometimes substitute for each other in a sequence. For example, phenylalanine occurs in motifs 1, 2, 3, and 4 as disclosed above, where it is believed to serve a hydrophobic function. The effects of amino acid sequence changes can be predicted by computer modeling using available software (e.g., the Insight II® viewer and homology modeling tools; MSI, San Diego, CA) or determined by alignment and analysis of crystal structures. See, Priestle et al., *EMBO J.* 7:339-343, 1988; Priestle et al., *Proc. Natl. Acad. Sci. USA* 86:9667-9671, 1989; Finzel et al., *J. Mol. Biol.* 209:779-791, 1989; Graves et al., *Biochem.* 29:2679-2684, 1990; Clore and Gronenbom, *J. Mol. Biol.* 221:47-53, 1991; Vigers et al., *J. Biol. Chem.* 269: 12874-12879, 1994; Schreuder et al., *Eur. J. Biochem.* 227:838-847, 1995; and Schreuder et al., *Nature* 386:194-200, 1997. A hydrophilicity profile of SEQ ID NO:2 is shown in Fig. 1. Those skilled in the art will recognize that hydrophobicity and hydrophilicity will be taken into account when designing alterations in the amino acid sequence of a zil1a4 polypeptide, so as not to disrupt the overall profile. Alignment of zil1a4 with other family members also provides guidance in selecting amino acid substitutions, particularly if information about the effects of amino acid substitutions in other family members is available. For example, alignment suggests that residue 200 (Glu) can be replaced with Lys, resulting in a change in activity from agonist to antagonist (Ju et al., *Proc. Natl. Acad. Sci.* USA 88:2658-2662, 1991; Oldfield et al., *Protein Eng.* 6:865-871, 1993). This variant of SEQ ID NO:2 (designated "zil1a4-E200K") is shown in SEQ ID NO: 14. A corresponding variant of SEQ ID NO:9 is shown in SEQ ID NO:15. It is in general preferred not to alter the sequences of the loops because these regions of the molecule are believed to be important for receptor binding, although residues 170, 171, 174, 186, 190, 199, and 200 of SEQ ID NO:2 may be substituted within the boundaries of the motifs defined herein. However, further alteration of the loops can be used to generate tools for investigation of receptor binding specificity and other aspects of interleukin biology.

Essential amino acids in the proteins of the present invention can be identified according to procedures known in the art, such as site-directed mutagenesis or alanine-scanning mutagenesis (Cunningham and Wells, *Science* 244, 1081-1085, 1989; Bass et al., *Proc. Natl. Acad. Sci. USA* 88:4498-4502, 1991). In the latter technique, single alanine mutations are introduced at every residue in the molecule, and the resultant mutant molecules are tested for biological activity or other properties to identify amino acid residues that are critical to the activity of the molecule.

Multiple amino acid substitutions can be made and tested using known methods of mutagenesis and screening, such as those disclosed by Reidhaar-Olson and Sauer (*Science* 241:53-57, 1988) or Bowie and Sauer (*Proc. Natl. Acad. Sci. USA* 86:2152-2156, 1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (e.g., Lowman et al., *Biochem.* 30:10832-10837, 1991; Ladner et al., U.S. Patent No. 5,223,409; Huse, WIPO Publication WO 92/06204) and region-directed mutagenesis (Derbyshire et al., *Gene* 46:145, 1986; Ner et al., *DNA* 7:127, 1988).

Variants of the disclosed zil1a4 DNA and polypeptide sequences can be generated through DNA shuffling as disclosed by Stemmer, *Nature* 370:389-391, 1994 and Stemmer, *Proc. Natl. Acad. Sci.* USA 91:10747-10751, 1994. Briefly, variant genes are generated by *in vitro* homologous recombination by random fragmentation of a parent gene followed by reassembly using PCR, resulting in randomly introduced point mutations. This technique can be modified by using a family of parent genes, such as allelic variants or genes from different species, to introduce additional variability into the process. Selection or screening for the desired activity, followed by additional iterations of mutagenesis and assay provides for rapid "evolution" of sequences by selecting for desirable mutations while simultaneously selecting against detrimental changes.

Mutagenesis methods as disclosed above can be combined with high volume or high-throughput screening methods to detect biological activity of zil1a4 variant proteins. Mutagenized DNA molecules that encode active zil1a4 proteins can be recovered from the host cells and rapidly sequenced using modem equipment. These methods allow the rapid determination of the importance of individual amino acid residues in a polypeptide of interest, and can be applied to polypeptides of unknown structure. Assays for IL-1 biological activity and receptor binding are known in the art.

Exemplary activity assays include mitogenesis assays in which IL-1 responsive cells (e.g., D10.N4.M Cells) are incubated in the presence of IL-1 or a test zil1a4 protein for 72 hours at 37°C in a 5% $CO_2$ atmosphere. IL-2 (and optionally IL-4) is added to the culture medium to enhance sensitivity and specificity of the assay. [$^3$H]thymidine is then added, and incubation is continued for six hours. The amount of label incorporated is indicative of agonist activity. See, Hopkins and Humphreys, *J. Immunol. Methods* 120:271-276, 1989; Greenfeder et al., *J. Biol. Chem.* 270:22460-22466, 1995. Zil1a4 stimulation of cell proliferation can also be measured using thymocytes cultured in a zil1a4 protein in combination with phytohemagglutinin. Proliferation is detected as $^3$H-thymidine incorporation or through the use of a colorimetric assay based on the metabolic breakdown of 3-(4,5-dimethylthiazol-2-yl)-2,5-diphenyl tetrazolium bromide (MTT) (Mosman, *J. Immunol. Meth.* 65: 55-63, 1983). Briefly, a solution of MTT is added to 100 µl of assay cells, and the cells are incubated at 37° C. After 4 hours, 200 µl of 0.04 N HCl in isopropanol is added, the solution is mixed, and the absorbance of the sample is measured at 570 nm.

Receptor binding can be measured by the competition binding method of Labriola-Tompkins et al., *Proc. Natl. Acad. Sci. USA* 88:11182-11186, 1991. Briefly, membranes pepared from EL-4 thymoma cells (Paganelli et al., *J. Immunol.* 138:2249-2253, 1987) are incubated in the presence of the test protein for 30 minutes at 37°C. Labeled IL-1α or IL-1β is then added, and the incubation is continued for 60 minutes. The assay is terminated by membrane filtration. The amount of bound label is determined by conventional means (e.g., γ counter). In an alternative assay, the ability of a zil1a4 protein to compete with labeled IL-1 for binding to cultured human dermal fibroblasts is measured according to the method of Dower et al. (*Nature* 324:266-268, 1986). Briefly, cells are incubated in a round-bottomed, 96-well plate in a suitable culture medium (e.g., RPMI 1640 containing 1% BSA, 0.1% Na azide, and 20 mM HEPES pH 7.4) at 8°C on a rocker platform in the presence of labeled IL-1. Various concentrations of zil1a4 protein are added. After the incubation (typically about two hours), cells are separated from unbound label by centrifuging 60-µl aliquots through 200 µl of phthalate oils in 400-µl polyethylene centrifuge tubes and excising the tips of the tubes with a razor blade as disclosed by Segal and Hurwitz, *J. Immunol*. 118:1338-1347, 1977. Receptor binding can also be measured using immobilized receptors or ligand-binding receptor fragments. For example, an immobilized IL-1 receptor can be exposed to labeled IL-1 and unlabeled test protein, whereby a reduction in IL-1 binding compared to a control is indicative of receptor-binding activity in the test protein. Within another format, a receptor or ligand-binding receptor fragment is immobilized on a biosensor (e.g., BIACore™, Pharmacia Biosensor, Piscataway, NJ) and binding is determined. Cloned cDNAs encoding mouse and human IL-1 receptors are disclosed by Dower et al., U.S. Patent No. 5,081,228. IL-1 antagonists will exhibit receptor binding but will exhibit essentially no activity in IL-1 activity assays or will reduce the IL-1-mediated response when combined with IL-1. In view of the low level of receptor occupancy required to produce a response to IL-1, a large excess of antagonist (typically a 10- to 1000-fold molar excess) may be necessary to neutralize IL-1 activity.

Cell activation can be assayed by measuring the expression of adhesion molecules or cytokines by responsive cells. Adhesion molecules such as ICAM, VCAM, and E-selectin on endothelial cells are known to be induced by IL-1β and other cytokines (Collins et al., *J. Biol. Chem*. 266:2466-2473, 1991; Iademarco et al., *J. Biol. Chem.* 267:1623-16329, 1992; Voraberger et al., *J. Immunol*. 147: 2777-2786, 1991). In various combinations, these and other molecules support leukocyte adhesion to the vessel wall and extravasation, which are key steps in the response to infection, inflammation, and tissue injury (Bevilacqua, *Annu. Rev. Immunol.* 11:767-804, 1993). Human umbilical vein endothelial cells (HUVECs) are harvested from umbilical cord veins and established in primary culture by methods that are well known in the art. Up-regulation of adhesion molecules can be measured by flow cytometric methods utilizing antibodies specific for these cell surface markers, by an ELISA-type assay, or by measuring the adherence of immune cells such as monocytes, T-cells, or neutrophils. Isolated cells can be used for this purpose, as can immortalized cells derived from these lineages, such as THP-1, U-937, HL-60, or Jurkat cells. Expression of these adhesion molecules can be measured in the presence or absence of IL-1β or other cytokines. T cells, natural killer (NK) cells, and monocytes/macrophages are the main producers of cytokines (Cassatella, *Immunology Today* 16:21-26, 1995). Cytokine release can be induced in these cells by various inflammatory stimuli and can be measured by assay as disclosed by Parrilo, *N. Engl. J. Med.* 328:1471-1477, 1993 or Eperon and Jungi, *J. Immunol. Methods.* 194:121-129, 1996. One such assay utilizes the monocytic cell line THP-1 induced by lipopolysaccharide (LPS). Cytokine release can be detected by immunoassay, such as an ELISA using antibodies specific for the cytokine of interest. Both activation and inhibition of cytokine release can be measured by these methods. See, Sandborg et al., *J. Immunol.* 155:5206-5212, 1995.

The proteins of the present invention can further comprise amino- or carboxyl-terminal extensions, such as an amino-terminal methionine residue, a small linker peptide of up to about 20-25 residues, or an affinity tag as disclosed above. Two or more affinity tags may be used in combination. Polypeptides comprising affinity tags can further comprise a polypeptide linker and/or a proteolytic cleavage site between the zit1a4 polypeptide and the affinity tag. Preferred cleavage sites include thrombin cleavage sites and factor Xa cleavage sites.

The present invention further provides a variety of other polypeptide fusions. For example, a zilla4 polypeptide can be prepared as a fusion to a dimerizing protein as disclosed in U.S. Patents Nos. 5,155,027 and 5,567,584. Preferred dimerizing proteins in this regard include immunoglobulin constant region domains. Immunoglobulin-zif1a4 polypeptide fusions can be expressed in genetically engineered cells to produce a variety of multimeric zil1a4 analogs. In addition, a zil1a4 polypeptide can be joined to another bioactive molecule, such as a cytokine, to provide a multifunctional molecule. One or more helices of a zil1a4 polypeptide can be joined to another cytokine to enhance or otherwise modify its biological properties. Auxiliary domains can be fused to zil1a4 polypeptides to target them to specific cells, tissues, or macromolecules (e.g., collagen). For example, a zil1a4 polypeptide or protein can be targeted to a predetermined cell type by fusing a zil1a4 polypeptide to a ligand that specifically binds to a receptor on the surface of the target cell. In this way, polypeptides and proteins can be targeted for therapeutic or diagnostic purposes. A zil1a4 polypeptide can be fused to two or more moieties, such as an affinity tag for purification and a targeting domain. Polypeptide fusions can also comprise one or more cleavage sites, particularly between domains. See, Tuan et al., *Connective Tissue Research* 34:1-9, 1996.

Polypeptide fusions of the present invention will generally contain not more than about 1,500 amino acid residues, preferably not more than about 1,200 residues, more preferably not more than about 1,000 residues, and will in many cases be considerably smaller. For example, a zil1a4 polypeptide of 218 residues (e.g., residues 1-218 of SEQ ID NO:2) can be fused to *E. coli* β-galactosidase (1,021 residues; see Casadaban et al., *J. Bacteriol.* 143:971-980, 1980), a 10-residue spacer, and a 4-residue factor Xa cleavage site to yield a polypeptide of 1,253 residues. In a second example, residues 60-218 of SEQ ID NO:14 can be fused to maltose binding protein (approximately 370 residues), a 4-residue cleavage site, and a 6-residue polyhistidine tag.

The present invention further provides polynucleotide molecules, including DNA and RNA molecules, encoding zit1a4 proteins. The polynucleotides of the present invention include the sense strand; the anti-sense strand; and the DNA as double-stranded, having both the sense and anti-sense strand annealed together by hydrogen bonds. Representative DNA sequences encoding zil1a4 proteins are set forth in SEQ ID NO:1, SEQ ID NO:8, and SEQ ID NO:13. Additional DNA sequences encoding these and other zil1a4 proteins can be readily generated by those of ordinary skill in the art based on the genetic code. Counterpart RNA sequences can be generated by substitution of U for T.

Those skilled in the art will readily recognize that, in view of the degeneracy of the genetic code, considerable sequence variation is possible among these polynucleotide molecules. SEQ ID NO:16 is a degenerate DNA sequence that encompasses all DNAs that encode the zil1a4 polypeptide of SEQ ID NO:2. SEQ ID NO:17 is a degenerate DNA sequence that encompasses all DNAs that encode the zil1a4 polypeptide of SEQ ID NO:14. SEQ ID NO:18 and SEQ ID NO:19 are degenerate DNA sequences that encompasses all DNAs that encode the zil1a4 polypeptides of SEQ ID NO:9 and SEQ ID NO:15, respectively. Those skilled in the art will recognize that the degenerate sequences of SEQ ID NO:16, SEQ ID NO:17, SEQ ID NO:18, and SEQ ID NO:19 also provide all RNA sequences encoding SEQ ID NO: 2, SEQ ID NO:14, SEQ ID NO:9, and SEQ ID NO:15 respectively, by substituting U for T. Thus, zil1a4 polypeptide-encoding polynucleotides comprising nucleotide 1 to nucleotide 654 of SEQ ID NO:16, nucleotide 1 to nucleotide 654 of SEQ ID NO:17, nucleotide 1 to nucleotide 471 of SEQ ID NO:18, nucleotide 1 to nucleotide 471 of SEQ ID NO:19, portions of these sequences encoding shorter zil1a4 proteins as disclosed above, and their respective RNA equivalents are contemplated by the present invention. Table 3 sets forth the one-letter codes used within SEQ ID NOS:16-19 to denote degenerate nucleotide positions. "Resolutions" are the nucleotides denoted by a code letter. "Complement" indicates the code for the complementary nucleotide(s). For example, the code Y denotes either C or T, and its complement R denotes A or G, A being complementary to T, and G being complementary to C.

TABLE 3

| Nucleotide | Resolution | Nucleotide | Complement |
|---|---|---|---|
| A | A | T | T |
| C | C | G | G |
| G | G | C | C |
| T | T | A | A |
| R | A\|G | Y | C\|T |
| Y | C\|T | R | A\|G |
| M | A\|C | K | G\|T |
| K | G\|T | M | A\|C |
| S | C\|G | S | C\|G |
| W | A\|T | W | A\|T |
| H | A\|C\|T | D | A\|G\|T |
| B | C\|G\|T | V | A\|C\|G |
| V | A\|C\|G | B | C\|G\|T |
| D | A\|G\|T | H | A\|C\|T |
| N | A\|C\|G\|T | N | A\|C\|G\|T |

The degenerate codons used in SEQ ID NOS:16-19, encompassing all possible codons for a given amino acid, are set forth in Table 4.

TABLE 4

| Amino Acid | One Letter Code | Codons | Degenerate Codon |
|---|---|---|---|
| Cys | C | TGC TGT | TGY |
| Ser | S | AGC AGT TCA TCC TCG TCT | WSN |
| Thr | T | ACA ACC ACG ACT | ACN |
| Pro | P | CCA CCC CCG CCT | CCN |
| Ala | A | GCA GCC GCG GCT | GCN |
| Gly | G | GGA GGC GGG GGT | GGN |
| Asn | N | AAC AAT | AAY |

TABLE 4 (continued)

| Amino Acid | One Letter Code | Codons | Degenerate Codon |
|---|---|---|---|
| Asp | D | GAC GAT | GAY |
| Glu | E | GAA GAG | GAR |
| Gln | Q | CAA CAG | CAR |
| His | H | CAC CAT | CAY |
| Arg | R | AGA AGG CGA CGC CGG CGT | MGN |
| Lys | K | AAA AAG | AAR |
| Met | M | ATG | ATG |
| Ile | I | ATA ATC ATT | ATH |
| Leu | L | CTA CTC CTG CTT TTA TTG | YTN |
| Val | V | GTA GTC GTG GTT | GTN |
| Phe | F | TTC TTT | TTY |
| Tyr | Y | TAC TAT | TAY |
| Trp | W | TGG | TGG |
| Ter | . | TAA TAG TGA | TRR |
| Asn\|Asp | B | | RAY |
| Glu\|Gln | Z | | SAR |
| Any | X | | NNN |

One of ordinary skill in the art will appreciate that some ambiguity is introduced in determining a degenerate codon, representative of all possible codons encoding each amino acid. For example, the degenerate codon for serine (WSN) can, in some circumstances, encode arginine (AGR), and the degenerate codon for arginine (MGN) can, in some circumstances, encode serine (AGY). A similar relationship exists between codons encoding phenylalanine and leucine. Thus, some polynucleotides encompassed by the degenerate sequence may encode variant amino acid sequences, but one of ordinary skill in the art can easily identify such variant sequences by reference to the amino acid sequences shown in SEQ ID NOS:2, 9, 14, and 15. Variant sequences can be readily tested for functionality as described herein.

Within preferred embodiments of the invention the isolated polynucleotides will hybridize to similar sized regions of SEQ ID NO:1, SEQ ID NO:8, or a sequence complementary thereto, under stringent conditions. In general, stringent conditions are selected to be about 5°C lower than the thermal melting point ($T_m$) for the specific sequence at a defined ionic strength and pH. The $T_m$ is the temperature (under defined ionic strength and pH) at which 50% of the target sequence hybridizes to a perfectly matched probe. Typical stringent conditions are those in which the salt concentration is up to about 0.03 M at pH 7 and the temperature is at least about 60°C.

One of ordinary skill in the art will also appreciate that different species can exhibit preferential codon usage. See, in general, Grantham et al., *Nuc. Acids Res.* 8:1893-912, 1980; Haas et al. *Curr. Biol.* 6:315-24, 1996; Wain-Hobson et al., *Gene* 13:355-64, 1981; Grosjean and Fiers, *Gene* 18:199-209, 1982; Holm, *Nuc. Acids Res.* 14:3075-87, 1986; and Ikemura, *J. Mol. Biol.* 158:573-97, 1982. Preferred codons for a particular species can be introduced into the polynucleotides of the present invention by a variety of methods known in the art. Introduction of preferred codon sequences into recombinant DNA can, for example, enhance production of the protein by making protein translation more efficient within a particular cell type or species. Therefore, the degenerate codon sequences disclosed in SEQ ID NOS:16-19 serve as templates for optimizing expression of polynucleotides in various cell types and species commonly used in the art and disclosed herein. Sequences containing preferred codons can be tested and optimized for expression in various host cell species, and tested for functionality as disclosed herein.

As previously noted, zil1a4 polynucleotides provided by the present invention include DNA and RNA. Methods for preparing DNA and RNA are well known in the art. In general, RNA is isolated from a tissue or cell that produces large amounts of zil1a4 RNA. Such tissues and cells are readily identified by Northern blotting (Thomas, *Proc. Natl. Acad. Sci. USA* 77:5201, 1980), and include bone marrow, fetal brain, fetal lung, lymph node, glioblastoma, monocytes, Daudi cells (a human Burkitt lymphoma cell line), and umbilical vein endothelial cells (HUVEC). Total RNA can be prepared using guanidine-HCl extraction followed by isolation by centrifugation in a CsCl gradient (Chirgwin et al., *Biochemistry* 18:52-94, 1979). Poly $(A)^+$ RNA is prepared from total RNA using the method of Aviv and Leder (*Proc. Natl. Acad. Sci. USA* 69:1408-12, 1972). Complementary DNA (cDNA) is prepared from poly$(A)^+$ RNA using known methods. In the altemative, genomic DNA can be isolated. Polynucleotides encoding zil1a4 polypeptides are then identified and isolated by, for example, hybridization or PCR.

The polynucleotides of the present invention can also be synthesized using automated equipment ("gene machines") according to methods known in the art. See, for example, Glick and Pasternak, Molecular Biotechnology Principles & Applications of Recombinant DNA, ASM Press, Washington, D.C., 1994; Itakura et al., *Annu. Rev. Biochem.* 53: 323-356, 1984; and Climie et al., *Proc. Natl. Acad. Sci. USA* 87:633-637, 1990.

The zil1a4 polynucleotide sequences disclosed herein can be used to isolate polynucleotides encoding other zil1a4 proteins. Such other proteins include alternatively spliced cDNAs (including cDNAs encoding secreted zil1a4 proteins) and counterpart polynucleotides from other species (orthologs). These orthologous polynucleotides can be used, *inter alia*, to prepare the respective orthologous proteins. Other species of interest include, but are not limited to, mammalian, avian, amphibian, reptile, fish, insect and other vertebrate and invertebrate species. Of particular interest are zil1a4 polynucleotides and proteins from other mammalian species, including non-human primate, murine, porcine, ovine, bovine, canine, feline, and equine polynucleotides and proteins. Orthologs of human zil1a4 can be cloned using information and compositions provided by the present invention in combination with conventional cloning techniques. For example, a cDNA can be cloned using mRNA obtained from a tissue or cell type that expresses zil1a4. Suitable sources of mRNA can be identified by PCR or by probing Northern blotting (Thomas, *Proc. Natl. Acad. Sci. USA* 77:5201, 1980) with probes designed from the sequences disclosed herein. A library is then prepared from mRNA of a positive tissue or cell line. A zil1a4-encoding cDNA can then be isolated by a variety of methods, such as by probing with a complete or partial human cDNA or with one or more sets of degenerate probes based on the disclosed sequences. Hybridization will generally be done under low stringency conditions, wherein washing is carried out in 1 x SSC with an initial wash at 40°C and with subsequent washes at 5°C higher intervals until background is suitably reduced. A cDNA can also be cloned using the polymerase chain reaction, or PCR (Mullis, U.S. Patent No. 4,683,202), using primers designed from the representative human zil1a4 sequence disclosed herein. Within an additional method, the cDNA library can be used to transform or transfect host cells, and expression of the cDNA of interest can be detected with an antibody to zil1a4 polypeptide. Similar techniques can also be applied to the isolation of genomic clones.

Preferred probes and primers are shown below in Table 5. These probes and primers are derived from least degenerate regions of an alignment of zil1a4 (SEQ ID NO:1), IL-1ra, IL-1$\alpha$, and IL-1$\beta$. The "consensus" and "complement" primers are useful for cloning both orthologs and paralogs, while the "zil1a4" primers will, in general, be more selective for orthologs. For each set of primers, the corresponding residues of human zil1a4 (SEQ ID NO:2) are indicated.

Table 5

| Sequence (5'→3') | Primer | SEQ ID NO: |
|---|---|---|
| - | - | 2 (residues 172-1779) |
| CAY CCN GGN TGG TTY AT | zil1a4 | 20 |
| YDY CCN RRN YKN TWY HT | Consensus | 21 |
| ADR WAN MRN YYN GGR HR | Complement | 22 |
| | | |
| - | - | 2 (residues 199-204) |
| ATH GAR TTY WSN TTY CA | zil1a4 | 23 |
| AYN RAN TTY HVN WTN YW | Consensus | 24 |
| WRN AWN BDR AAN TYN RT | Complement | 25 |
| | | |
| - | - | 2 (residues 59-64) |
| AAR TTY WSN ATH CAY GA | zil1a4 | 26 |
| RMN TKY HBN HTN HRN GA | Consensus | 27 |
| TCN YDN ADN VDR MAN KY | Complement | 28 |
| | | |
| - | - | 2 (residues 117-122) |
| GAR TTY TGY YTN TAY TG | zil1a4 | 29 |
| VAN HTN TRY BTN WVN KS | Consensus | 30 |
| SMN BWN AVR YAN ADN TB | Complement | 31 |

Those skilled in the art will recognize that the sequence disclosed in SEQ ID NO:1 represents a single allele of human zil1a4, and that natural variation, including allelic variation and alternative splicing (e.g., as shown in SEQ ID NO:8), is expected to occur. Allelic variants of this sequence can be cloned by probing cDNA or genomic libraries from different individuals according to standard procedures. Allelic variants of the DNA sequence shown in SEQ ID NO:1, including those containing silent mutations and those in which mutations result in amino acid sequence changes, are within the scope of the present invention, as are proteins which are allelic variants of SEQ ID NO:2. cDNAs generated from alternatively spliced mRNAs, which retain the immune modulating activity of zil1a4 are included within the scope of the present invention, as are polypeptides encoded by such cDNAs and mRNAs. For example, the protein shown in SEQ ID NO:2 is believed to represent a cytoplasmically expressed form of the protein in view of the lack of a signal peptide sequence. There is expected to be an alternately spliced form of the DNA which includes a secretory signal sequence.

Allelic variants and splice variants of these sequences can be cloned by probing cDNA or genomic libraries from different individuals or tissues according to standard procedures known in the art.

For any zil1a4 polypeptide, including variants and fusion proteins, one of ordinary skill in the art can readily generate a fully degenerate polynucleotide sequence encoding that variant using the information set forth in Tables 3 and 4, above. Moreover, those of skill in the art can use standard software to devise zil1a4 variants based upon the nucleotide and amino acid sequences described herein. The present invention thus provides a computer-readable medium encoded with a data structure that provides at least one of the following sequences: SEQ ID NO:1, SEQ ID NO:2, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, and portions thereof. Suitable forms of computer-readable media include magnetic media and optically-readable media. Examples of magnetic media include a hard or fixed drive, a random access memory (RAM) chip, a floppy disk, digital linear tape (DLT), a disk cache, and a ZIP™ disk. Optically readable media are exemplified by compact discs (e.g., CD-read only memory (ROM), CD-rewritable (RW), and CD-recordable), and digital versatile/video discs (DVD) (e.g., DVD-ROM, DVD-RAM, and DVD+RW).

The proteins of the present invention, including full-length proteins, variant proteins, and fusion proteins can be produced in genetically engineered host cells according to conventional techniques. Suitable host cells are those cell types that can be transformed or transfected with exogenous DNA and grown in culture, and include bacteria, fungal cells, and cultured higher eukaryotic cells. Eukaryotic cells, particularly cultured cells of multicellular organisms, are preferred. Techniques for manipulating cloned DNA molecules and introducing exogenous DNA into a variety of host cells are disclosed by Sambrook et al., *Molecular Cloning: A Laboratory Manual,* 2nd ed., Cold Spring Harbor Laboratory Press, Cold Spring Harbor, NY, 1989, and Ausubel et al., eds., *Current Protocols in Molecular Biology,* Green and Wiley and Sons, NY, 1993.

In general, a DNA sequence encoding a zil1a4 protein is operably linked to other genetic elements required for its expression, generally including a transcription promoter and terminator, within an expression vector. The vector will also commonly contain one or more selectable markers and one or more origins of replication, although those skilled in the art will recognize that within certain systems selectable markers may be provided on separate vectors, and replication of the exogenous DNA may be provided by integration into the host cell genome. Selection of promoters, terminators, selectable markers, vectors and other elements is a matter of routine design within the level of ordinary skill in the art. Many such elements are described in the literature and are available through commercial suppliers.

To direct a zil1a4 protein into the secretory pathway of a host cell, a secretory signal sequence (also known as a leader sequence, prepro sequence or pre sequence) is provided in the expression vector. The secretory signal sequence may be that of a zil1a4 gene, or may be derived from another secreted protein (e.g., t-PA) or synthesized *de novo.* The secretory signal sequence is operably linked to the zil1a4 DNA sequence, i.e., the two sequences are joined in the correct reading frame and positioned to direct the newly sythesized polypeptide into the secretory pathway of the host cell. Secretory signal sequences are commonly positioned 5' to the DNA sequence encoding the polypeptide of interest, although certain signal sequences may be positioned elsewhere in the DNA sequence of interest (see, e. g., Welch et al., U.S. Patent No. 5,037,743; Holland et al., U.S. Patent No. 5,143,830). In the alternative, a zil1a4 protein is expressed cytoplasmically and is isolated after lysing the host cells.

Cultured mammalian cells are suitable hosts for use within the present invention. Methods for introducing exogenous DNA into mammalian host cells include calcium phosphate-mediated transfection (Wigler et al., *Cell* 14: 725, 1978; Corsaro and Pearson, *Somatic Cell Genetics* 7:603, 1981: Graham and Van der Eb, *Virology* 52:456, 1973), electroporation (Neumann et al., *EMBO J.* 1:841-845, 1982), DEAE-dextran mediated transfection (Ausubel et al., *ibid*.), and liposome-mediated transfection (Hawley-Nelson et al., *Focus* 15:73, 1993; Ciccarone et al., *Focus* 15:80, 1993). The production of recombinant polypeptides in cultured mammalian cells is disclosed by, for example, Levinson et al., U.S. Patent No. 4,713,339; Hagen et al., U.S. Patent No. 4,784,950; Palmiter et al., U.S. Patent No. 4,579,821; and Ringold, U.S. Patent No. 4,656,134. Suitable cultured mammalian cells include the COS-1 (ATCC No. CRL 1650), COS-7 (ATCC No. CRL 1651), BHK (ATCC No. CRL 1632), BHK 570 (ATCC No. CRL 10314), 293 (ATCC No. CRL 1573; Graham et al., *J. Gen. Virol.* 36:59-72, 1977) and Chinese hamster ovary (e.g. CHO-K1; ATCC No. CCL 61) cell lines. Additional suitable cell lines are known in the art and available from public depositories such as the American Type Culture Collection, Rockville, Maryland. In general, strong transcription promoters are preferred, such as promoters from SV-40 or cytomegalovirus. See, e.g., U.S. Patent No. 4,956,288. Other suitable promoters include those from metallothionein genes (U.S. Patent Nos. 4,579,821 and 4,601,978) and the adenovirus major late promoter.

Drug selection is generally used to select for cultured mammalian cells into which foreign DNA has been inserted. Such cells are commonly referred to as "transfectants". Cells that have been cultured in the presence of the selective agent and are able to pass the gene of interest to their progeny are referred to as "stable transfectants." An exemplary selectable marker is a gene encoding resistance to the antibiotic neomycin. Selection is carried out in the presence of a neomycin-type drug, such as G-418 or the like. Selection systems can also be used to increase the expression level of the gene of interest, a process referred to as "amplification." Amplification is carried out by culturing transfectants in the presence of a low level of the selective agent and then increasing the amount of selective agent to select for cells that produce high levels of the products of the introduced genes. An exemplary amplifiable selectable marker is dihydrofolate reductase, which confers resistance to methotrexate. Other drug resistance genes (e.g. hygromycin resistance, multi-drug resistance, puromycin acetyltransferase) can also be used.

Other higher eukaryotic cells can also be used as hosts, including insect cells, plant cells and avian cells. The use of *Agrobacterium rhizogenes* as a vector for expressing genes in plant cells has been reviewed by Sinkar et al., *J. Biosci. (Bangalore)* 11:47-58, 1987. Transformation of insect cells and production of foreign polypeptides therein is disclosed by Guarino et al., U.S. Patent No. 5,162,222 and WIPO publication WO 94/06463.

Insect cells can be infected with recombinant baculovirus, commonly derived from *Autographa californica* nuclear polyhedrosis virus (AcNPV). See, King and Possee, The Baculovirus Expression System: A Laboratory Guide, London, Chapman & Hall; O'Reilly et al., Baculovirus Expression Vectors: A Laboratory Manual, New York, Oxford University Press., 1994; and Richardson, Ed., Baculovirus Expression Protocols. Methods in Molecular Biology, Humana Press, Totowa, NJ, 1995. Recombinant baculovirus can also be produced through the use of a transposon-based system described by Luckow et al. (*J. Virol*. 67:4566-4579, 1993). This system, which utilizes transfer vectors, is commercially available in kit form (Bac-to-Bac™ kit; Life Technologies, Rockville, MD). The transfer vector (e.g., pFastBac1™; Life Technologies) contains a Tn7 transposon to move the DNA encoding the protein of interest into a baculovirus genome maintained in *E. coli* as a large plasmid called a "bacmid." See, Hill-Perkins and Possee, *J. Gen. Virol*. 71:971-976, 1990; Bonning et al., *J. Gen. Virol*. 75:1551-1556, 1994; and Chazenbalk and Rapoport, *J. Biol. Chem.* 270:1543-1549, 1995. For protein production, the recombinant virus is used to infect host cells, typically a cell line derived from the fall armyworm, *Spodoptera frugiperda* (e.g., Sf9 or Sf21 cells) or *Trichoplusia ni* (e.g., High Five™ cells; Invitrogen, Carlsbad, CA). See, in general, Glick and Pasternak, Molecular Biotechnology: Principles and Applications of Recombinant DNA, ASM Press, Washington, D.C., 1994. See also, U.S. Patent No. 5,300,435. Procedures used are generally described in available laboratory manuals (e.g., King and Possee, *ibid*.; O'Reilly et al., *ibid*.; Richardson, *ibid*.).

Fungal cells, including yeast cells, can also be used within the present invention. Yeast species of particular interest in this regard include *Saccharomyces cerevisiae, Pichia pastoris*, and *Pichia methanolica.* Methods for transforming *S. cerevisiae* cells with exogenous DNA and producing recombinant polypeptides therefrom are disclosed by, for example, Kawasaki, U.S. Patent No. 4,599,311; Kawasaki et al., U.S. Patent No. 4,931,373; Brake, U.S. Patent No. 4,870,008; Welch et al., U.S. Patent No. 5,037,743; and Murray et al., U.S. Patent No. 4,845,075. Transformed cells are selected by phenotype determined by the selectable marker, commonly drug resistance or the ability to grow in the absence of a particular nutrient (e.g., leucine). A preferred vector system for use in *Saccharomyces cerevisiae* is the *POT1* vector system disclosed by Kawasaki et al. (U.S. Patent No. 4,931,373), which allows transformed cells to be selected by growth in glucose-containing media. Suitable promoters and terminators for use in yeast include those from glycolytic enzyme genes (see, e.g., Kawasaki, U.S. Patent No. 4,599,311; Kingsman et al., U.S. Patent No. 4,615,974; and Bitter, U.S. Patent No. 4,977,092) and alcohol dehydrogenase genes. See also U.S. Patents Nos. 4,990,446; 5,063,154; 5,139,936 and 4,661,454. Transformation systems for other yeasts, including *Hansenula polymorpha, Schizosaccharomyces pombe, Kluyveromyces lactis, Kluyveromyces fragilis, Ustilago maydis, Pichia pastoris, Pichia methanolica, Pichia guillennondii* and *Candida maltosa* are known in the art. See, for example, Gleeson et al., J. *Gen. Microbiol*. 132:3459-3465, 1986; Cregg, U.S. Patent No. 4,882,279; and Raymond et al., *Yeast* 14, 11-23, 1998. Aspergillus cells may be utilized according to the methods of McKnight et al., U.S. Patent No. 4,935,349. Methods for transforming *Acremonium chrysogenum* are disclosed by Sumino et al., U.S. Patent No. 5,162,228. Methods for transforming Neurospora are disclosed by Lambowitz, U.S. Patent No. 4,486,533. Production of recombinant proteins in *Pichia methanolica* is disclosed in U.S. Patents No. 5,716,808, 5,736,383, 5,854,039, and 5,888,768; and WIPO publications WO 99/14347 and WO 99/14320.

Prokaryotic host cells, including strains of the bacteria *Escherichia coli, Bacillus* and other genera are also useful host cells within the present invention. Techniques for transforming these hosts and expressing foreign DNA sequences cloned therein are well known in the art (see, e.g., Sambrook et al., ibid.). When expressing a zil1a4 polypeptide in bacteria such as *E. coli,* the polypeptide may be retained in the cytoplasm, either in soluble form or as insoluble granules, or may be directed to the pefiplasmic space by a bacterial secretion sequence. When the protein is present as insoluble granules, the cells are lysed, and the granules are recovered and denatured using, for example, guanidine isothiocyanate or urea. The denatured polypeptide can then be refolded and dimerized by diluting the denaturant, such as by dialysis against a solution of urea and a combination of reduced and oxidized glutathione, followed by dialysis against a buffered saline solution. In the latter case, the polypeptide can be recovered from the periplasmic space in a soluble and functional form by disrupting the cells (by, for example, sonication or osmotic shock) to release the contents of the periptasmic space and recovering the protein, thereby obviating the need for denaturation and refolding.

Transformed or transfected host cells are cultured according to conventional procedures in a culture medium containing nutrients and other components required for the growth of the chosen host cells. A variety of suitable media, including defined media and complex media, are known in the art and generally include a carbon source, a nitrogen source, essential amino acids, vitamins and minerals. Media may also contain such components as growth factors or serum, as required. The growth medium will generally select for cells containing the exogenously added DNA by, for example, drug selection or deficiency in an essential nutrient which is complemented by the selectable marker carried on the expression vector or co-transfected into the host cell.

Using the methods disclosed above, one of ordinary skill in the art can identify and/or prepare a variety of zil1a4 proteins having immunomodulatory activity. Such polypeptides can also include additional polypeptide segments as generally disclosed above.

It is preferred to purify the proteins of the present invention to ≥80% purity, more preferably to ≥90% purity, even more preferably ≥95% purity, and particularly preferred is a pharmaceutically pure state, that is greater than 99.9% pure with respect to contaminating macromolecules, particularly other proteins and nucleic acids, and free of infectious and pyrogenic agents. Preferably, a purified protein is substantially free of other proteins, particularly other proteins of animal origin. Zil1a4 proteins (including fusion proteins) are purified by conventional protein purification methods, typically by a combination of chromatographic techniques. See, in general, Affinity Chromatography: Principles & Methods, Pharmacia LKB Biotechnology, Uppsala, Sweden, 1988; and Scopes, Protein Purification: Principles and Practice, Springer-Verlag, New York, 1994. Proteins comprising a polyhistidine affinity tag (typically about 6 histidine residues) are purified by affinity chromatography on an immobilized nickel or cobalt resin. See, for example, Houchuli et al., *Bio/Technol.* 6: 1321-1325, 1988. Proteins comprising a glu-glu tag can be purified by immunoaffinity chromatography according to conventional procedures. See, for example, Grussenmeyer et al., *ibid.* Maltose binding protein fusions are purified on an amylose column according to methods known in the art.

Zil1a4 proteins of the present invention can be used to modulate inflammation and other immunological processes. Of particular interest is the reduction of inflammation by antagonist forms of zil1a4 (e.g., SEQ ID NO:14). Thus, certain zil1a4 proteins may be used to treat or prevent chronic and acute inflammatory diseases such as arthritis, including rheumatoid arthritis, osteoarthritis, and Lyme arthritis, and psoriasis; to reduce tissue damage after ischemia; and to treat septic shock, graft-versus-host disease, and leukemia. Other conditions that may be modulated by zil1a4 proteins include cancer, anemia, inflammatory bowel disease, autoimmunity, acute and chronic neuropathologies, shock, acute respiratory disease syndrome, restenosis, and AIDS. As used herein, the terms "treat" and "treatment" will be understood to include the reduction of symptoms as well as effects on the underlying disease processes. Antagonists may have *in vivo* activity like that of IL-1 receptor antagonist (IL-1ra), which has shown beneficial effects in clinical trials directed to the treatment of rheumatoid arthritis (Campion et al., *Arthritis & Rheumatism* 39:1092-1101, 1996), graft-versus-host disease (Antin et al., Blood 84:1342-1348, 1994), septic shock (Fisher et al., *JAMA* 271: 1836-1843, 1994), and leukemia (Dinarello, Blood 87:2095-2147, 1996). Experimental data also suggest that antagonism of IL-1 activity will prove beneficial in the treatment of inflammatory bowel disease (including Crohn's disease and ulcerative colitis) (reviewed by Hendel et al., *Exp. Opin. Invest. Drugs* 5:843-850, 1996; see also, Cominelli et al., *Gastroenterology* 103:65-71, 1992), insulin-dependent diabetes mellitus (reviewed by Mandrup-Poulsen et al., *Cytokine* 5:185-191, 1993; see also, Dayer-Metroz et al., *Eur. J. Clin. Inv.* 22:A50, 1992), acute pancreatitis (Norman et al., *Ann. Surg.* 221:625-634, 1995), glomerulonephritis (Lan et al., *Kidney Int.* 47:1303-1309, 1995), and cerebral ischemia (Relton et al., *Exp. Neurology* 138:206-213, 1996; Loddick et al., *Biochem. Biophys. Res. Comm.* 234: 211-215, 1997). Agonists may promote wound healing in view of the effects of IL-1 on growth factor secretion and cell proliferation or be useful in the treatment of infections, in particular gastrointestinal infections.

Zil1a4 proteins can be tested in animal models of disease. Animal models of psoriasis include the analysis of histological alterations in adult mouse tail epidermis (Hofbauer et al, *Brit. J. Dermatol.* 118:85-89, 1988; Bladon et al., *Arch Dermatol. Res.* 277:121-125, 1985). In this model, anti-psoriatic activity is indicated by the induction of a granular layer and orthokeratosis in areas of scale between the hinges of the tail epidermis. Typically, a topical ointment is applied daily for seven consecutive days, then the animal is sacrificed, and tail skin is examined histologically. An additional model is provided by grafting psoriatic human skin to congenitally athymic (nude) mice (Krueger et al., *J. Invest. Dermatol.* 64:307-312, 1975). Such grafts have been shown to retain the characteristic histology for up to eleven weeks. As in the mouse tail model, the test composition is applied to the skin at predetermined intervals for a period of one to several weeks, at which time the animals are sacrificed and the skin grafts examined histologically. A third model has been disclosed by Fretland et al. *(Inflammation* 14:727-739, 1990; incorporated herein by reference). Briefly, inflammation is induced in guinea pig epidermis by topically applying phorbol ester (phorbol-12-myristate-13-acetate; PMA), typically at ca. 2 g/ml in acetone, to one ear and vehicle to the contralateral ear. Test compounds are applied concurrently with the PMA, or may be given orally. Histological analysis is performed at 96 hours after application of PMA. This model duplicates many symptoms of human psoriasis, including edema, inflammatory cell diapedesis and infiltration, high $LTB_4$ levels and epidermal proliferation. Cerebral ischemia can be studied in a rat model as disclosed by Relton et al. (*ibid.*) and Loddick et al. (*ibid*.). Wound-healing models include the linear skin incision model of Mustoe et al. (*Science* 237:1333, 1987). In a typical procedure, a 6-cm incision is made in the dorsal pelt of an adult rat, then closed with wound clips. Test substances and controls (in solution, gel, or powder form) are applied before primary closure. It is preferred to limit administration to a single application, although additional applications can be made on succeeding days by careful injection at several sites under the incision. Wound breaking strength is evaluated between 3 and 21 days post wounding. In a second model, multiple, small, full-thickness excisions are made on the ear of a rabbit. The cartilage in the ear splints the wound, removing the variable of wound contraction from the evaluation of closure. Experimental treatments and controls are applied. The geometry and anatomy of the wound site allow for reliable quantification of cell ingrowth and epithelial migration, as well as quantitative analysis of the biochemistry of the wounds (e.g., collagen content). See, Mustoe et al., *J. Clin. Invest*. 87:694, 1991. The rabbit ear model can be modified to create an ischemic wound environment, which more closely resembles the clinical situation (Ahn et al., *Ann. Plast. Surg.* 24:17, 1990). Within a third model, healing of partial-thickness skin wounds in pigs or guinea pigs is evaluated (LeGrand et al., *Growth Factors* 8:307, 1993). Experimental treatments are applied daily on or under dressings. Seven days after wounding, granulation tissue thickness is determined. This model is preferred for dose-response studies, as it is more quantitative than other *in vivo* models of wound healing. A full thickness excision model can also be employed. Within this model, the epidermis and dermis are removed down to the panniculus camosum in rodents or the subcutaneous fat in pigs. Experimental treatments are applied topically on or under a dressing, and can be applied daily if desired. The wound closes by a combination of contraction and cell ingrowth and proliferation. Measurable endpoints include time to wound closure, histologic score, and biochemical parameters of wound tissue. Impaired wound healing models are also known in the art (e.g., Cromack et al., *Surgery* 113:36, 1993; Pierce et al., *Proc. Natl. Acad. Sci. USA* 86:2229, 1989; Greenhalgh et al., *Amer. J. Pathol*. 136:1235, 1990). Delay or prolongation of the wound healing process can be induced pharmacologically by treatment with steroids, irradiation of the wound site, or by concomitant disease states (e.g., diabetes). Linear incisions or full-thickness excisions are most commonly used as the experimental wound. Endpoints are as disclosed above for each type of wound. Subcutaneous implants can be used to assess compounds acting in the early stages of wound healing (Broadley et al., *Lab. Invest.* 61:571, 1985; Sprugel et al., *Amer. J. Pathol.* 129: 601, 1987). Implants are prepared in a porous, relatively non-inflammatory container (e.g., polyethylene sponges or expanded polytetrafluoroethylene implants filled with bovine collagen) and placed subcutaneously in mice or rats. The interior of the implant is empty of cells, producing a "wound space" that is well-defined and separable from the preexisting tissue. This arrangement allows the assessment of cell influx and cell type as well as the measurement of vasculogenesis/angiogenesis and extracellular matrix production. As disclosed by Jovcic et al. (*Leukemia* 10:546-549, 1996), the proliferation of hematopoietic progenitor cells is responsive to levels of IL-1. Effects of IL-1 and agonists and antagonists thereof can be measured using sub-lethally irradiated test animals (e.g., mice). Briefly, a test compound is administered directly to irradiated mice, or bone marrow cells are preincubated with the test compound and transplanted into irradiated mice. Proliferative and anti-proliferative effects are seen as changes in colony formation as compared to controls. Induction of tolerance to ischemia can be measured in Mongolian gerbils as disclosed by Hallenbeck, *Neurology* 49(Suppl. 4):S5-S9, 1997.

Expression of zil1a4 proteins in animals provides models for further study of the biological effects of overproduction or inhibition of protein activity *in vivo.* Zil1a4-encoding polynucleotides can be introduced into test animals, such as mice, using viral vectors or naked DNA, or transgenic animals can be produced.

One *in vivo* approach for assaying proteins of the present invention utilizes viral delivery systems. Exemplary viruses for this purpose include adenovirus, herpesvirus, retroviruses, vaccinia virus, and adeno-associated virus (AAV). Adenovirus, a double-stranded DNA virus, is currently the best studied gene transfer vector for delivery of heterologous nucleic acids. For review, see Becker et al., *Meth. Cell Biol*. 43:161-89, 1994; and Douglas and Curiel, *Science & Medicine* 4:44-53, 1997. The adenovirus system offers several advantages. Adenovirus can (i) accommodate relatively large DNA inserts; (ii) be grown to high-titer; (iii) infect a broad range of mammalian cell types; and (iv) be used with many different promoters including ubiquitous, tissue specific, and regulatable promoters. Because adenoviruses are stable in the bloodstream, they can be administered by intravenous injection. When intravenously administered to intact animals, adenovirus primarily targets the liver. If the adenoviral delivery system has an E1 gene deletion, the virus cannot replicate in the host cells. However, the host's tissue (e.g., liver) will express and process (and, if a signal sequence is present, secrete) the heterologous protein. Secreted proteins will enter the circulation in the highly vascularized liver, and effects on the infected animal can be determined.

In another embodiment, a zil1a4 gene can be introduced in a retroviral vector as described, for example, by Anderson et al., U.S. Patent No. 5,399,346; Mann et al., *Cell* 33:153, 1983; Temin et al., U.S. Patent No. 4,650,764; Temin et al., U.S. Patent No. 4,980,289; Markowitz et al., *J. Virol.* 62:1120, 1988; Temin et al., U.S. Patent No. 5,124,263; Dougherty et al., WIPO publication WO 95/07358; and Kuo et al., *Blood* 82:845, 1993.

In an alternative method, the vector can be introduced by "lipofection" *in vivo* using liposomes. Synthetic cationic lipids can be used to prepare liposomes for *in vivo* transfection of a gene encoding a marker (Felgner et al., *Proc. Natl. Acad. Sci. USA* 84:7413-7, 1987; Mackey et al., *Proc. Natl. Acad. Sci. USA* 85:8027-31, 1988). The use of lipofection to introduce exogenous genes into specific organs *in vivo* has certain practical advantages, including molecular targeting of liposomes to specific cells. Directing transfection to particular cell types is particularly advantageous in a tissue with cellular heterogeneity, such as the pancreas, liver, kidney, and brain. Lipids may be chemically coupled to other molecules for the purpose of targeting. Targeted peptides (e.g., hormones or neurotransmitters), proteins such as antibodies, or non-peptide molecules can be coupled to liposomes chemically.

Within another embodiment target cells are removed from the the animal, and the DNA is introduced as a naked DNA plasmid. The transformed cells are then re-implanted into the body of the animal. Naked DNA vectors can be introduced into the desired host cells by methods known in the art, e.g., transfection, electroporation, microinjection, transduction, cell fusion, DEAE dextran, calcium phosphate precipitation, use of a gene gun or use of a DNA vector transporter. See, e.g., Wu et al., J. *Biol. Chem.* 267:963-7, 1992; Wu et al., *J. Biol. Chem.* 263:14621-4, 1988.

Transgenic animals, engineered to express a zil1a4 gene, and animals that exhibit a complete absence of zil1a4 gene function, referred to as "knockout mice" (Snouwaert et al., *Science* 257:1083, 1992), can be generated (Lowell et al., *Nature* 366:740-42, 1993). See also, Brinster et al., *Proc. Natl. Acad. Sci. USA* 85: 836-840, 1988; Palmiter et al., *Proc. Natl. Acad. Sci. USA* 88: 478-482, 1991; Whitelaw et al., *Transgenic Res.* 1: 3-13, 1991; and WIPO publications WO 89/01343 and WO 91/02318). Polynucleotides used in generating transgenic animals that express a zil1a4 gene will preferably contain one or more introns; genomic sequences are thus preferred.

Antisense methodology can be used to inhibit zil1a4 gene transcription to examine the effects of such inhibition *in vivo*. Polynucleotides that are complementary to a segment of a zil1a4-encoding polynucleotide (e.g., a polynucleotide as set froth in SEQ ID NO:1) are designed to bind to zil1a4-encoding mRNA and to inhibit translation of such mRNA.

The activity of zil1a4 proteins can be measured with a silicon-based biosensor microphysiometer that measures the extracellular acidification rate or proton excretion associated with receptor binding and subsequent physiologic cellular responses. An exemplary such device is the Cytosensor™ Microphysiometer manufactured by Molecular Devices, Sunnyvale, CA. A variety of cellular responses, such as cell proliferation, ion transport, energy production, inflammatory response, regulatory and receptor activation, and the like, can be measured by this method. See, for example, McConnell et al., *Science* 257:1906-1912, 1992; Pitchford et al., *Meth. Enzymol.* 228:84-108, 1997; Arimilli et al., *J. Immunol. Meth*. 212:49-59, 1998; and Van Liefde et al., *Eur. J. Pharmacol*. 346:87-95, 1998. Antagonists of zil1a4 can be identified by exposing the cells to zil1a4 protein in the presence and absence of the test compound, whereby a reduction in zil1a4-stimulated activity is indicative of antagonist activity in the test compound.

For pharmaceutical use, the proteins of the present invention are formulated for local, including topical, or parenteral, including intravenous, subcutaneous, or intraperitoneal delivery according to conventional methods. Intravenous administration will be by injection or infusion. In many instances it will be beneficial to administer the protein by infusion or multiple injections per day over a period of several days to several weeks, sometimes preceded by a bolus injection. In general, pharmaceutical formulations will include a zil1a4 protein in combination with a pharmaceutically acceptable vehicle, such as saline, buffered saline, 5% dextrose in water or the like. Formulations may further include one or more excipients, preservatives, solubilizers, buffering agents, albumin to prevent protein loss on vial surfaces, etc. Methods of formulation are well known in the art and are disclosed, for example, in Remington: The Science and Practice of Pharmacy, Gennaro, ed., Mack Publishing Co., Easton, PA, 19th ed., 1995.

As noted above, inhibition of IL-1 activity requires a large molar excess of antagonist. Doses of zil1a4 antagonist proteins will in general be quite large, particularly when treating life-threatening conditions. IL-1ra appears safe in high doses. Thus, doses of zil1a4 antagonist proteins will range from as low as 10 mg per patient per day to as high as 100 mg or more per hour infused over a period days. Doses of IL-1ra found to be efficacious in clinical studies include 70 mg per patient per day in rheumatoid arthritis and up to 3,400 mg per patient per day in graft-versus-host disease. The exact dose will be determined by the clinician according to accepted standards, taking into account the nature and severity of the condition to be treated, patient traits, etc. Determination of dose is within the level of ordinary skill in the art. The proteins may be administered for acute treatment, over one week or less, but will often be used in treatment of chronic conditions requiring administration over several weeks to several months or longer. In general, a therapeutically effective amount of a zil1a4 protein is an amount sufficient to produce a clinically significant improvement in one or more standard indicators appropriate to the treated condition. Therapeutic endpoints will be apparent to those skilled in the art.

Zil1a4 proteins, both agonist and antagonist, can be used as standards in assays of IL-1 and IL-1 inhibitor.

Such assays can comprise any of a number of standard formats, include radioreceptor assays and ELISAs. Zil1a4 protein standards can be prepared in labeled form using a radioisotope, enzyme, fluorophore, or other compound that produces a detectable signal. The proteins can be packaged in kit form, such kits comprising one or more vials containing the zilla4 protein and, optionally, a diluent, an antibody, a labeled binding protein, etc.

Assay kits can also be used in the research laboratory to detect IL-1 and IL-1 inhibitor activities produced by cultured cells or test animals.

Zilla4 proteins are also useful as research reagents. For example, zit1a4 agonist proteins can be used as cell culture components to promote the growth of IL-1 responsive cells, including fibroblasts, smooth muscle cells, and mesangial cells. Agonists can be combined with IL-3 and other cytokines to expand $CD34^+$ peripheral blood cells, and with IL-3 and IL-6 to promote the proliferation of stem cells.

Zilla4 proteins and epitope-bearing portions thereof can be use to generate antibodies that specifically bind to zil1a4. An "epitope" is a region of a protein to which an antibody can bind. See, for example, Geysen et al., *Proc. Natl. Acad. Sci. USA* 81:3998-4002, 1984. Epitopes can be linear or conformational, the latter being composed of discontinuous regions of the protein that form an epitope upon folding of the protein. Linear epitopes are generally at least 6 amino acid residues in length. Relatively short synthetic peptides that mimic part of a protein sequence are routinely capable of eliciting an antiserum that reacts with the partially mimicked protein. See, Sutcliffe et al., *Science* 219:660-666, 1983. Antibodies that recognize short, linear epitopes are particularly useful in analytic and diagnostic applications that employ denatured protein, such as Western blotting (Tobin, *Proc. Natl. Acad. Sci. USA* 76:4350-4356, 1979), or in the analysis of fixed cells or tissue samples. Antibodies to linear epitopes are also useful for detecting fragments of zil1a4 in, for example, body fluids or cell culture media.

Antigenic, epitope-bearing polypeptides contain a sequence of at least six, preferably at least nine, more preferably from 15 to about 30 contiguous amino acid residues of a zil1a4 protein (e.g., SEQ ID NO:2). Polypeptides comprising a larger portion of a zil1a4 protein, i.e. from 30 to 50 residues up to the entire sequence, can also be used. It is preferred that the amino acid sequence of the epitope-bearing polypeptide is selected to provide substantial solubility in aqueous solvents, that is the sequence includes relatively hydrophilic residues, and hydrophobic residues are substantially avoided. See the attached Fig. 1. Sequences containing proline residues are preferred. Particularly preferred such regions include residues 122-127, 123-128, and 147-152 of SEQ ID NO:2.

As used herein, the term "antibodies" includes polyclonal antibodies, monoclonal antibodies, antigen-binding fragments thereof such as $F(ab')_2$ and Fab fragments, single chain antibodies, and the like, including genetically engineered antibodies. Non-human antibodies can be humanized by grafting only non-human CDRs onto human framework and constant regions, or by incorporating the entire non-human variable domains (optionally "cloaking" them with a human-like surface by replacement of exposed residues, wherein the result is a "veneered" antibody). In some instances, humanized antibodies may retain non-human residues within the human variable region framework domains to enhance proper binding characteristics. Through humanizing antibodies, biological half-life may be increased, and the potential for adverse immune reactions upon administration to humans is reduced. One skilled in the art can generate humanized antibodies with specific and different constant domains (i.e., different Ig subclasses) to facilitate or inhibit various immune functions associated with particular antibody constant domains. Alternative techniques for generating or selecting antibodies useful herein include *in vitro* exposure of lymphocytes to a zil1a4 protein, and selection of antibody display libraries in phage or similar vectors (for instance, through use of immobilized or labeled zil1a4 polypeptide). Antibodies are defined to be specifically binding if they bind to a zil1a4 protein with an affinity at least 10-fold greater than the binding affinity to control (non-zil1a4) polypeptide. It is preferred that the antibodies exhibit a binding affinity ($K_a$) of $10^6$ $M^{-1}$ or greater, preferably $10^7$ $M^{-1}$ or greater, more preferably $10^8$ $M^{-1}$ or greater, and most preferably $10^9$ $M^{-1}$ or greater. The affinity of a monoclonal antibody can be readily determined by one of ordinary skill in the art (see, for example, Scatchard, *Ann. NY Acad. Sci*. 51: 660-672, 1949).

Methods for preparing polyclonal and monoclonal antibodies are well known in the art (see for example, Hurrell, J. G. R., Ed., *Monoclonal Hybridoma Antibodies: Techniques and Applications,* CRC Press, Inc., Boca Raton, FL, 1982). As would be evident to one of ordinary skill in the art, polyclonal antibodies can be generated from a variety of warm-blooded animals such as horses, cows, goats, sheep, dogs, chickens, rabbits, mice, and rats. The immunogenicity of a zil1a4 protein may be increased through the use of an adjuvant such as alum (aluminum hydroxide) or Freund's complete or incomplete adjuvant. Polypeptides useful for immunization also include fusion polypeptides, such as fusions of a zilla4 protein or a portion thereof with an immunoglobulin polypeptide or with maltose binding protein. The polypeptide immunogen may be a full-length molecule or a portion thereof. If the polypeptide portion is "hapten-like", such portion may be advantageously joined or linked to a macromolecular carrier (such as keyhole limpet hemocyanin (KLH), bovine serum albumin (BSA) or tetanus toxoid) for immunization.

A variety of assays known to those skilled in the art can be used to detect antibodies that specifically bind to a zil1a4 protein. Exemplary assays are described in detail in *Antibodies: A Laboratory Manual*, Harlow and Lane (Eds.), Cold Spring Harbor Laboratory Press, 1988. Representative examples of such assays include concurrent immunoelectrophoresis, radioimmunoassays, radio-immunoprecipitations, enzyme-linked immunosorbent assays (ELISA), dot blot assays, Western blot assays, inhibition or competition assays, and sandwich assays.

Antibodies to zil1a4 may be used for affinity purification of zil1a4 proteins; within diagnostic assays for determining circulating levels of zil1a4 proteins; for detecting or quantitating soluble zil1a4 protein as a marker of underlying pathology or disease; for immunolocalization within whole animals or tissue sections, including immunodiagnostic applications; for immunohistochemistry; for screening expression libraries; and for other uses that will be evident to those skilled in the art. For certain applications, including *in vitro* and *in vivo* diagnostic uses, it is advantageous to employ labeled antibodies. Suitable direct tags or labels include radionuclides, enzymes, substrates, cofactors, inhibitors, fluorescent markers, chemiluminescent markers, magnetic particles and the like; indirect tags or labels may feature use of biotin-avidin or other complement/anti-complement pairs as intermediates.

The present invention also provides polynucleotide reagents for diagnostic use. For example, a zil1a4 gene, a probe comprising zil1a4 DNA or RNA, or a subsequence thereof can be used to determine if the zil1a4 gene is present on chromosome 2 of a human patient or if a mutation has occurred. Detectable chromosomal aberrations at the zil1a4 gene locus include, but are not limited to, aneuploidy, gene copy number changes, insertions, deletions, restriction site changes and rearrangements. Such aberrations can be detected using polynucleotides of the present invention by employing molecular genetic techniques, such as restriction fragment length polymorphism (RFLP) analysis, short tandem repeat (STR) analysis employing PCR techniques, and other genetic linkage analysis techniques known in the art (Sambrook et al., *ibid*.; Ausubel et. al., *ibid*.; A.J. Marian, *Chest* 108:255-265, 1995).

The invention is further illustrated by the following non-limiting examples.

## EXAMPLES

### Example 1

An expressed sequence tag (ESTs) was identified in a public database by its sequence homology to the IL-1 family of proteins. A second EST was identified in a private database by its overlap with the first EST.

Analysis of a clone corresponding to the second EST indicated the presence of an intron at its 5' end. Based on a consensus sequence derived from the the two ESTs, 6 primers were designed. One pair of these primers (zc20,038, SEQ ID NO:32 and zc20,039, SEQ ID NO:33) was used for a PCR-based tissue survey. A total of 23 human cDNA libraries were prepared from various tissues using a commercially available kit (Marathon™ cDNA Amplification Kit from Clontech Laboratories, Inc., Palo Alto, CA). These libraries were used as PCR templates. 3 µl of each cDNA library (diluted 1/100), 20 pmoles of each of oligonucleotide primers zc20,038 (SEQ ID NO:32) and zc20,039 (SEQ ID NO:33), and 1 U of *Taq* DNA polymerase (Ex Taq™ polymerase; PanVera, Madison, WI) were used in 25-µl reactions. The reactions were run as follows: 94°C for 2 minutes; then 35 cycles of 94°C for 20 seconds, 63°C for 30 seconds, 72°C for 30 seconds; and ending with a 2-minute incubation at 72°C. PCR products were run on a gel. The right sized bands were found in bone marrow, Daudi, fetal brain, fetal lung, glioblastoma, HUVEC, lymph node, and monocyte, but not in $CD4^+$ cells, $CD8^+$ cells, kidney, liver, islet, pancreas, salivary gland, skeletal muscle, spleen, stomach, testis, and uterus.

### Example 2

On the basis of the tissue distribution information above, zil1a4 cDNA was cloned using nested PCR with 5' and 3' RACE.

5' RACE was performed on bone marrow, Daudi, fetal brain, fetal lung, glioblastoma, HUVEC, lymph node, monocyte, and testis. 3 µl of each cDNA library (diluted 1/100), 20 pmoles of each of oligonucleotide primers zc9739 (SEQ ID NO:34) and zc20,042 (SEQ ID NO:35), and 1 U of a 1:1 mixture of *Taq* DNA polymerase and anti-Taq antibody (TaqStart™; Clontech Laboratories) (*ExTaq*/Taq antibody 1:1) were used in 25-µl reactions. The reactions were run as follows: 94°C for 2 minutes; then 5 cycles of 94°C for 20 seconds, 69°C for 1 minute; 30 cycles of 94°C for 20 seconds, 64°C for 30 seconds, 72°C for 1 minute; and ending with a 2-minute incubation at 72°C. 1 µl of each 1/30 diluted first PCR product was used as template for a nested PCR. 20 pmoles each of oligonucleotide primers ZC9719 (SEQ ID NO:36) and ZC20,043 (SEQ ID NO:37) and 1 U of *ExTaq*/Taq antibody 1:1 were used in 25-µl reactions. The reactions were run as follows: 94°C for 2 minutes; then 5 cycles of 94°C for 20 seconds, 67°C for 1 minute; 30 cycles of 94°C for 20 seconds, 64°C for 30 seconds, 72°C for 1 minute; and ending with a 1-minute incubation at 72°C. The PCR products were run on an agarose gel. Obvious bands were obtained from Daudi, fetal brain, fetal lung, glioblastoma, and lymph node. Bands were then purified with a spin column containing a silica gel membrane (QIAquick™ Gel Extraction Kit; Qiagen, Inc., Valencia, CA) and sequenced. Sequence results from the PCR bands extended the 5' end of the first EST, but disagreed with the second EST clone, which was believed to contain an intron at the 5' end.

Because the 3' end sequence of the second EST clone differed from the first EST, 3' RACE was performed. cDNAs (prepared as disclosed above) from three tissues (Daudi, fetal brain, and lymph node) were used as templates.

3 µl of each cDNA (diluted 1/100), 20 pmoles of each of oligonucleotide primers zc9739 (SEQ ID NO:34) and zc20,040 (SEQ ID NO:38), and 1 U of *ExTaq*/Taq antibody 1:1 were used in 25-µl reactions. The reactions were run as follows: 94°C for 2 minutes; then 5 cycles of 94°C for 20 seconds, 70°C for 30 seconds; 25 cycles of 94°C for 20 seconds, 64°C for 30 seconds, 72°C for 30 seconds; and ending with a 2-minute incubation at 72°C. One µl of a 1/30 dilution of each of the PCR products was used as template for a nested PCR. 20 pmoles each of oligonucleotide primers ZC9719 (SEQ ID NO:36) and ZC20,041 (SEQ ID NO:39) and 1 U of *ExTaq*/Taq antibody 1:1 were used in 25-µl reactions. The reactions were run as follows: 94°C for 2 minutes; then 5 cycles of 94°C for 20 seconds, 68°C for 30 seconds; 30 cycles of 94°C for 20 seconds, 64°C for 30 seconds, 72°C for 30 seconds; and ending with a 2-minute incubation at 72°C. The PCR products were run on an agarose gel. Obvious bands were obtained in Daudi and fetal brain. Bands were then purified with a spin column containing a silica gel membrane (QIAquick™ Gel Extraction Kit; Qiagen, Inc., Valencia, CA) and sequenced. Sequence results from the PCR bands extended the 3'end of the original EST, but disagreed with the second EST clone. The consensus DNA sequence, a composite of six 5' RACE products, two ESTs, and two 3' RACE products, is shown in SEQ ID NO:1.

Example 3

The entire zil1a4 coding region was inserted into the vector pCR®2.1 (Invitrogen, Carlsbad, CA). PCR was performed using 3 µl of each 1/100 diluted cDNA from Daudi, fetal brain, fetal lung, glioblastoma, lymph node and testis; 20 pmoles each of oligonucleotide primers ZC20,308 (SEQ ID NO:40) and ZC20,309 (SEQ ID NO:41); and 1 U of a 4:1:4 mixture of *Taq* DNA polymerase, *Pfu* DNA polymerase (Stratagene, La Jolla, CA), and anti-Taq antibody (TaqStart™; Clontech Laboratories) (*ExTaq*/Pfu/Taq 4:1:4) in 25-µl reaction mixtures. The reactions were run as follows: 94°C for 2 minutes; then 35 cycles of 94°C for 20 seconds, 62°C for 30 seconds, 72°C for 30 seconds; and ending with a 4-minute incubation at 72°C. PCR products were run on a gel, and bands were purified with a spin column containing a silica gel membrane (QIAquick™ Gel Extraction Kit; Qiagen, Inc., Valencia, CA). Six µl of gel purified PCR fragment was ligated into 2 µl of pCR®2.1 vector (Invitrogen) using a commercially available cloning kit (TA Cloning® Kit; Invitrogen) and incubated at 16°C overnight. 1 µl of the ligation mixture was mixed with 10 µl of *E. coli* host cells (Electromax DH10B™ cells; obtained from Life Technologies, Inc., Gaithersburg, MD), electroporated, then mixed with 400 µl LB, and shaken at 37°C for 30 minutes. 100 µl of cells was plated on LB/amp plates with 80 µl of 0.1 M IPTG and 80 µl of 20 mg/ml X-gal, and the plates were incubated at 37°C overnight. White colonies were screened by PCR using the same conditions as above, except that the template was bacterial colonies instead of cDNA, and the enzyme mixture was *ExTaq*/Taq antibody 1:1. Colonies with inserts were selected for sequencing.

Example 4

Northern blots were performed using a series of Northern blots (Human Multiple Tissue Blots I, II and III, Human RNA Master blot and Human Fetal MTN II from Clontech Laboratories, Inc., Palo Alto, CA). The probe was made from a gel-purified PCR product using the second EST clone as a template. PCR conditions were as disclosed in Example 1. DNA was radioactively labeled with a commercially available kit (Rediprime™ II random-prime labeling system; Amersham Corp., Arlington Heights, IL) according to the manufacturer's directions. The probe was purified using a commercially available push column (NucTrap® column; Stratagene, La Jolla, CA; see U.S. Patent No. 5,336,412). A commercial hybridization solution (ExpressHyb™ Hybridization Solution; Clontech Laboratories, Inc., Palo Alto, CA) was used for prehybridization and as a hybridizing solution for the blots. Hybridization took place ovemight at 65°C, and the blots were then washed three times in 2X SSC and 0.05% SDS at 55°C, followed by two washes in 0.1X SSC and 0.1 % SDS at 55°C. No obvious signal was found in most of the tissue, except a weak signal was detected in testis and placenta on the RNA Master blot.

Example 5

An alternatively spliced zil1a4 cDNA was cloned from a human fetal lung library. The DNA and amino acid sequences of this clone are shown in SEQ ID NO:8 and SEQ ID NO:9. The clone lacked 183 bp as compared to SEQ ID NO:1.

Example 6

Zil1a4 was mapped to human chromosome 2 using the commercially available version of the Stanford G3 Radiation Hybrid Mapping Panel (Research Genetics, Inc., Huntsville, AL).This panel contains PCRable DNAs from each of 83 radiation hybrid clones of the whole human genome, plus two control DNAs (the RM donor and the A3 recipient). A publicly available WWW server (http://shgc-www.stanford.edu) allows chromosomal localization of markers. For the mapping of Zil1a4, 20-µl reaction mixtures were set up in a PCRable 96-well microtiter plate (Stratagene, La Jolla, CA) and used in a thermal cycler (RoboCycler® Gradient 96; Stratagene, La Jolla, CA). Each of the 85 reaction mixtures consisted of 2 µl buffer (10X KlenTaq PCR reaction buffer, Clontech Laboratories, Inc., Palo Alto, CA), 1.6 µl dNTPs mix (2.5 mM each, PERKIN-ELMER, Foster City, CA), 1 µl sense primer (SEQ ID NO:43), 1 µl antisense primer (SEQ ID NO:44), 2 µl of a density increasing agent and tracking dye (RediLoad, Research Genetics, Inc., Huntsville, AL), 0.4 µl of a commercially available DNA polymerase/antibody mix (50X Advantage™ KlenTaq Polymerase Mix, Clontech Laboratories, Inc.), 25 ng of DNA from an individual hybrid clone or control and x µl $ddH_2O$ for a total volume of 20 µl. The mixtures were overlaid with an equal amount of mineral oil and sealed. PCR was run for an initial 5-minute denaturation at 94°C; 35 cycles of 45 seconds denaturation at 94°C, 45 seconds annealing at 60°C, and 75 seconds extension at 72°C; followed by a final extension of 7 minutes at 72°C. The reaction products were separated by electrophoresis on a 2% agarose gel. The results showed linkage of the zil1a4 gene to the human chromosome 2 framework marker AFMa037xf1 with a LOD score of 12.5 and at a distance of 8.60 cR_10000 from the marker. The use of surrounding genes/markers positioned Zil1a4 in the 2q14 chromosomal region.

Example 7

Recombinant human zil1a4 is produced in *E. coli* using a $His_6$ tag/maltose binding protein (MBP) double affinity fusion system as generally disclosed by Pryor and Leiting, *Prot. Expr. Pur.* 10:309-319, 1997. A thrombin cleavage site is placed at the junction between the affinity tag and zil1a4 sequences.

The fusion construct is assembled in the vector pTAP98, which comprises sequences for replication and selection in *E. coli* and yeast, the *E. coli* tac promoter, and a unique SmaI site just downstream of the MBP-$His_6$-thrombin site coding sequences. The zil1a4 cDNA (SEQ ID NO:1) is amplified by PCR using primers each comprising 40 bp of sequence homologous to vector sequence and 25 bp of sequence that anneals to the cDNA. The reaction is run using Pwo DNA polymerase (Boehringer Mannheim, Indianapolis, IN) for 30 cycles of 94°C, 30 seconds; 60°C, 60 seconds; and 72°C, 60 seconds. One microgram of the resulting fragment is mixed with 100 ng of SmaI-cut pTAP98, and the mixture is transformed into yeast to assemble the vector by homologous recombination (Oldenburg et al., *Nucl. Acids.* Res. 25:451-452, 1997). $Ura^+$ transformants are selected.

Plasmid DNA is prepared from yeast transformants and transformed into *E. coli* MC1061. Pooled plasmid DNA is then prepared from the MC1061 transformants by the miniprep method after scraping an entire plate. Plasmid DNA is analyzed by restriction digestion using NcoI and EcoRI.

*E. coli* strain BL21 is used for expression of zil1a4. Cells are transformed by electroporation and grown on minimal glucose plates containing casamino acids and ampicillin. For larger-scale production cells are grown in liquid media containing ampicillin. After one hour at 37°C, IPTG is added to a final concentration of 1 mM, and the cells are grown for an additional 2-3 hours at 37°C. Cells are disrupted using glass beads, and extracts are prepared according to conventional methods.

Example 8

Recombinant zil1a4 fusion protein is purified by affinity chromatography. Immobilized cobalt resin (Talon® resin; Clontech Laboratories, Inc., Palo Alto, CA) is equilibrated in binding buffer. One ml of packed resin per 50 mg protein is combined with a clarified protein solution in a tube, and the tube is capped and sealed, then placed on a rocker overnight at 4°C. The resin is then pelleted by centrifugation at 4°C and washed three times with binding buffer. Protein is eluted with binding buffer containing 0.2 M imidazole. The resin and elution buffer are mixed for at least one hour at 4°C, the resin is pelleted, and the supematant is removed. An aliquot is analyzed by gel electrophoresis, and concentration is estimated. Amylose resin is equilibrated in amylose binding buffer (20 mM Tris-HCl, pH 7.0, 100 mM NaCl, 10 mM EDTA) and combined with the supematant from the cobalt resin at a ratio of 2 mg fusion protein per ml of resin. Binding and washing steps are carried out as disclosed above. Protein is eluted with amylose binding buffer containing 10 mM maltose using as small a volume as possible to minimize the need for subsequent concentration. The eluted protein is analyzed by gel electrophoresis and staining with Coomassie blue using a BSA standard, and by Western blotting using an anti-MBP antibody.

**Claims**

1. An isolated protein comprising a sequence of amino acid residues selected from the group consisting of:

   residues 60 through 218 of SEQ ID NO:7; and
   residues 1 through 157 of SEQ ID NO:10.

2. The isolated protein of claim 1 wherein the amino acid residue corresponding to residue number 200 of SEQ ID NO:2 is Lys.

3. The isolated protein of claim 1 wherein the amino acid residue corresponding to residue number 200 of SEQ ID NO:2 is Asp.

4. The isolated protein of claim 1 wherein the amino acid residue corresponding to residue number 200 of SEQ ID NO:2 is Glu.

5. The isolated protein of claim 1 wherein said protein comprises residues 60 through 218 of SEQ ID NO:11.

6. The isolated protein of claim 5 wherein said protein comprises residues 60 through 218 of SEQ ID NO:14 or SEQ ID NO:2.

7. The isolated protein of claim 1 wherein said protein comprises residues 1 through 218 of SEQ ID NO:11 or residues 1 through 157 of SEQ ID NO:12

8. The isolated protein of claim 7 wherein said protein comprises:

   residues 1 through 218 of SEQ ID NO:14;
   residues 1 through 218 of SEQ ID NO:2;
   residues 1 through 157 of SEQ ID NO:15; or
   residues 1 through 157 of SEQ ID NO:9.

9. The isolated protein of any claims 1-8 wherein said protein is from 157 to 1500 amino acid residues in length.

10. The isolated protein of any of claims 1-9, further comprising an affinity tag.

11. An isolated polynucleotide encoding a protein according to any of claims 1-10.

12. The isolates polynucleotide of claim 11, which is DNA.

13. An expression vector comprising the following operably linked elements:

   (a) a transcription promoter;
   (b) a DNA segment encoding a protein according to any of claims 1-10; and
   (c) a transcription terminator.

14. The expression vector of claim 13 further comprising a secretory signal sequence operably linked to said DNA segment.

15. A cultured sell comprising an expression vector according to claim 14.

16. A method of making a protein comprising: culturing a cell according to claim 15 under conditions wherein said DNA segment is expressed; and recovering the protein encoded by said DNA segment.

17. Use of a protein according to any of claims 1-10 in combination with a pharmaceutically acceptable vehicle in the preparation of a medicament for modulating an immune response in an animal.

## Patentansprüche

1. Isoliertes Protein, umfassend eine Sequenz von Aminosäureresten, die ausgewählt sind aus der Gruppe bestehend aus:

   den Resten 60 bis 218 von SEQ ID NO:7; und
   den Resten 1 bis 157 von SEQ ID NO:10.

2. Isoliertes Protein nach Anspruch 1, wobei der Aminosäurerest, der dem Rest Nummer 200 von SEQ ID NO:2 entspricht, Lys ist.

3. Isoliertes Protein nach Anspruch 1, wobei der Aminosäurerest, der dem Rest Nummer 200 von SEQ ID NO:2 entspricht, Asp ist.

4. Isoliertes Protein nach Anspruch 1, wobei der Aminosäurerest, der dem Rest Nummer 200 von SEQ ID NO:2 entspricht, Glu ist.

5. Isoliertes Protein nach Anspruch 1, wobei das Protein die Reste 60 bis 218 von SEQ ID NO:11 umfasst.

6. Isoliertes Protein nach Anspruch 5, wobei das Protein die Reste 60 bis 218 von SEQ ID NO:14 oder SEQ ID NO: 2 umfasst.

7. Isoliertes Protein nach Anspruch 1, wobei das Protein die Reste 1 bis 218 von SEQ ID NO:11 oder die Reste 1 bis 157 von SEQ ID NO:12 umfasst.

8. Isoliertes Protein nach Anspruch 7, wobei das Protein umfasst:

   die Reste 1 bis 218 von SEQ ID NO:14;
   die Reste 1 bis 218 von SEQ ID NO:2;
   die Reste 1 bis 157 von SEQ ID NO:15; oder
   die Reste 1 bis 157 von SEQ ID NO:9.

9. Isoliertes Protein nach irgendeinem der Ansprüche 1-8, wobei das Protein eine Länge von 157 bis 1500 Aminosäureresten hat.

10. Isoliertes Protein nach irgendeinem der Ansprüche 1-9, welches ferner einen Affinitäts-Tag umfasst.

11. Isoliertes Polynucleotid, welches ein Protein nach irgendeinem der Ansprüche 1-10 codiert.

12. Isoliertes Polynucleotid nach Anspruch 11, welches DNA ist.

13. Expressionsvektor, umfassend die folgenden funktionsfähig miteinander verknüpften Elemente:

   (a) einen Transkriptionspromotor;
   (b) ein DNA-Segment, das ein Protein nach irgendeinem der Ansprüche 1-10 codiert;
   (c) einen Transkriptionsterminator.

14. Expressionsvektor nach Anspruch 13, welcher ferner eine funktionsfähig mit dem DNA-Segment verknüpfte Sekretionssignalsequenz umfasst.

15. Kultivierte Zelle, umfassend einen Expressionsvektor nach Anspruch 14.

16. Verfahren zur Herstellung eines Proteins, welches umfasst: Kultivieren einer Zelle nach Anspruch 15 unter Bedingungen, bei denen das DNA-Segment exprimiert wird; und Rückgewinnen des von dem DNA-Segment codierten Proteins.

17. Verwendung eines Proteins nach irgendeinem der Ansprüche 1-10 in Kombination mit einem pharmazeutisch verträglichen Trägerstoff zur Herstellung eines Medikaments zum Modulieren einer Immunantwort bei einem Lebewesen.

**Revendications**

1. Protéine isolée comprenant une séquence de résidus d'acides aminés choisis dans le groupe constitué par :

   les résidus 60 à 218 de SEQ ID n° 7 ; et
   les résidus 1 à 157-de SEQ ID n° 10.

2. Protéine isolée selon la revendication 1,
   **caractérisée en ce que**
   le résidu d'acide aminé correspondant au numéro de résidu 200 de SEQ ID n° 2 est Lys.

3. Protéine isolée selon la revendication 1,
   **caractérisée en ce que**
   le résidu d'acide aminé correspondant au numéro de résidu 200 de SEQ ID n° 2 est Asp.

4. Protéine isolée selon la revendication 1,

**caractérisée en ce que**
le résidu d'acide aminé correspondant au numéro de résidu 200 de SEQ ID n° 2 est Glu.

**5.** Protéine isolée selon la revendication 1,
**caractérisée en ce que**
la protéine comprend les résidus 60 à 218 de SEQ ID n° 11.

**6.** Protéine isolée selon la revendication 5,
**caractérisée en ce que**
la protéine comprend les résidus 60 à 218 de SEQ ID n°14 ou de SEQ ID n° 2.

**7.** Protéine isolée selon la revendication 1,
**caractérisée en ce que**
la protéine comprend les résidus 1 à 218 de SEQ ID n° 11 ou les résidus 1 à 157 de SEQ ID n° 12.

**8.** Protéine isolée selon la revendication 7,
**caractérisée en ce que**
la protéine comprend :

les résidus 1 à 218 de SEQ ID n° 14 ;
les résidus 1 à 218 de SEQ ID n° 2 ;
les résidus 1 à 157 de SEQ ID n° 15 ; ou
les résidus 1 à 157 de SEQ ID n° 9.

**9.** Protéine isolée selon l'une quelconque des revendications 1 à 8,
**caractérisée en ce que**
la protéine a une longueur de 157 à 1500 résidus d'acides aminés.

**10.** Protéine isolée selon l'une quelconque des revendications 1 à 9, comprenant en outre un marqueur d'affinité.

**11.** Polynucléotide isolé codant pour une protéine selon l'une quelconque des revendications 1 à 10.

**12.** Polynucléotide isolé selon la revendication 11, qui est l'ADN.

**13.** Vecteur d'expression comprenant les éléments suivants liés de manière fonctionnelle :

(a) un promoteur transctiptionnel ;
(b) un segment d'ADN codant pour une protéine selon l'une quelconque des revendications 1 à 10 ; et
(c) un terminateur transcriptionnel.

**14.** Vecteur d'expression selon la revendication 13, comprenant en outre une séquence de signaux sécrétoires liée de manière fonctionnelle au segment d'ADN.

**15.** Cellule cultivée comprenant un vecteur d'expression selon la revendication 14.

**16.** Procédé de préparation d'une protéine comprenant :

- la culture d'une cellule selon la revendication 15 dans des conditions au cours desquelles le segment d'ADN est exprimé ; et
- la récupération de la protéine codée par le segment d'ADN.

**17.** Utilisation d'une protéine selon l'une quelconque des revendications 1 à 10, combinée à un véhicule pharmaceutiquement acceptable dans la préparation d'un médicament pour moduler une réponse immune chez un animal.

Hydrophobic -2 -1 0 1 2 Hydrophilic

| | | |
|---|---|---|
| 1 | 0.33 | M |
| 2 | 0.47 | S |
| 3 | 0.36 | F |
| 4 | 0.67 | V |
| 5 | 0.56 | G |
| 6 | 0.94 | E |
| 7 | 0.12 | N |
| 8 | 0.08 | S |
| 9 | 0.08 | G |
| 10 | 0.58 | V |
| 11 | 1.33 | K |
| 12 | 0.67 | M |
| 13 | 1.38 | G |
| 14 | 1.88 | S |
| 15 | 2.33 | E |
| 16 | 2.33 | D |
| 17 | 1.83 | w |
| 18 | 2.03 | E |
| 19 | 1.37 | K |
| 20 | 0.70 | D |
| 21 | -0.10 | E |
| 22 | -0.10 | P |
| 23 | 0.40 | Q |
| 24 | 0.37 | C |
| 25 | 0.45 | C |
| 26 | 0.62 | L |
| 27 | 0.97 | E |
| 28 | 0.47 | D |
| 29 | -0.33 | P |
| 30 | 0.17 | A |
| 31 | 0.25 | G |
| 32 | 0.25 | S |
| 33 | 0.20 | P |
| 34 | 0.25 | L |
| 35 | 0.25 | E |
| 36 | -0.25 | P |
| 37 | -0.32 | G |
| 38 | -0.53 | P |
| 39 | -0.50 | S |
| 40 | -0.97 | L |
| 41 | -0.92 | P |
| 42 | -1.00 | T |
| 43 | -1.00 | M |
| 44 | -0.73 | N |
| 45 | -0.77 | F |
| 46 | 0.15 | V |
| 47 | 0.15 | H |
| 48 | 0.73 | T |
| 49 | 0.83 | S |

Fig. 1A

```
50    0.48                      P =====
51    0.52                      K =====
52    0.02                      V
53    0.77                      K ========
54    0.77                      N ========
55    0.32                      L ===
56    0.67                      N =======
57    0.33                      P ===
58    0.25                      K ===
59    0.25                      K ===
60   -0.22                   == F
61    0.70                      S =======
62    0.97                      I ==========
63    1.77                      H ==================
64    1.60                      D ================
65    0.80                      Q ========
66    0.52                      D =====
67   -0.28                  === h
68   -0.10                    = K
69   -0.55               ====== V
70   -0.30                  === L
71    0.03                      V
72   -0.02                      L
73   -0.02                      D
74   -0.60               ====== S
75   -0.90            ========= G
76   -0.90            ========= N
77   -0.43                 ==== L
78    0.37                      I ====
79    0.70                      A =======
80    0.40                      V ====
81    0.35                      P ====
82    0.85                      D =========
83    0.35                      K ====
84    0.35                      N ====
85    0.02                      Y
86   -0.02                      I
87   -0.13                    = R
88   -0.72              ======= P
89   -1.02           ========== E
90   -1.60     ================ I
91   -1.87  =================== F
92   -1.40       ============== F
93   -1.28        ============= A
94   -1.15         ============ L
95   -0.80             ======== A
96   -0.80             ======== s
97   -0.18                   == S
98   -0.32                  === L
99    0.48                      S =====
100   0.93                      S =========
101   0.88                      A =========
102   1.02                      S ==========
103   0.97                      A ==========
```

**Fig. 1B**

| | | | | |
|---|---|---|---|---|
| 104 | 0.75 | | E | ======== |
| 105 | -0.05 | | K | |
| 106 | -0.85 | ========= | G | |
| 107 | -1.42 | ============== | S | |
| 108 | -1.72 | ================= | P | |
| 109 | -1.67 | ================= | I | |
| 110 | -0.87 | ========= | L | |
| 111 | -0.57 | ====== | L | |
| 112 | 0.23 | | g | == |
| 113 | 0.38 | | V | ==== |
| 114 | 0.47 | | S | ===== |
| 115 | 0.12 | | K | = |
| 116 | -0.77 | ======== | G | |
| 117 | -0.93 | ========= | E | |
| 118 | -0.93 | ========= | F | |
| 119 | -0.02 | | C | |
| 120 | 0.65 | | L | ======= |
| 121 | 1.45 | | Y | ============== |
| 122 | 1.83 | | C | ================== |
| 123 | 2.03 | | D | ==================== |
| 124 | 1.58 | | K | ================ |
| 125 | 1.40 | | D | ============== |
| 126 | 0.90 | | K | ========= |
| 127 | 0.45 | | G | ===== |
| 128 | 0.15 | | Q | == |
| 129 | 0.15 | | S | == |
| 130 | -0.20 | == | h | |
| 131 | -0.02 | | P | |
| 132 | 0.48 | | S | ===== |
| 133 | 0.93 | | L | ========= |
| 134 | 1.73 | | Q | ================= |
| 135 | 1.40 | | L | ============== |
| 136 | 1.48 | | K | =============== |
| 137 | 1.48 | | K | =============== |
| 138 | 0.68 | | E | ======= |
| 139 | 0.10 | | K | = |
| 140 | -0.48 | ===== | L | |
| 141 | -0.15 | = | M | |
| 142 | 0.57 | | K | ====== |
| 143 | 0.57 | | L | ====== |
| 144 | 0.92 | | A | ========= |
| 145 | 0.92 | | A | ========= |
| 146 | 1.50 | | Q | =============== |
| 147 | 1.97 | | K | ==================== |
| 148 | 1.47 | | E | =============== |
| 149 | 0.55 | | S | ====== |
| 150 | 0.20 | | A | == |
| 151 | -0.13 | = | R | |
| 152 | -1.02 | ========== | R | |
| 153 | -1.02 | ========== | P | |
| 154 | -1.10 | =========== | F | |
| 155 | -0.65 | ======= | I | |
| 156 | -0.60 | ====== | F | |
| 157 | -0.18 | == | Y | |

Fig. 1C

```
158   0.25                        R ===
159  -0.42                   ==== A
160  -0.30                    === Q
161  -0.55                 ====== V
162  -0.60                 ====== G
163  -0.10                      = S
164  -0.10                      = w
165  -0.02                        N
166  -0.13                      = M
167   0.00                        L
168   0.30                        E ===
169  -0.20                     == S
170  -0.82               ======== A
171  -1.15           ============ A
172  -1.37         ============== H
173  -1.45         ============== P
174  -2.02   ==================== G
175  -1.97   ==================== W
176  -1.57       ================ F
177  -1.12            =========== I
178  -0.98             ========== C
179  -0.78               ======== t
180   0.28                        S ===
181   0.23                        C ==
182   0.15                        N ==
183   0.12                        C =
184   0.03                        N
185  -0.07                      = E
186  -0.07                      = P
187   0.43                        V ====
188   0.27                        G ===
189   0.77                        V ========
190   1.05                        T ===========
191   1.62                        D ================
192   1.62                        K ================
193   1.03                        F ==========
194   1.15                        E ============
195   1.15                        N ============
196   0.70                        R =======
197   0.25                        K ===
198  -0.67                ======= H
199  -0.55                 ====== I
200  -0.25                    === E
201  -1.00             ========== F
202  -0.75               ======== S
203  -0.30                    === F
204   0.03                        Q
205   0.50                        P =====
206   0.28                        V ===
207   0.58                        C ======
208   0.86                        K =========
209   0.52                        A =====
210   1.22                        E ============
211   0.58                        M ======
```

**Fig. 1D**

```
212   0.96                                         S ==========
213   1.52                                         P ==============
214   0.00                                         S
215   0.00                                         E
216   0.00                                         V
217   0.00                                         S
218   0.00                                         D
            ----|---------|---------  |  ---------|---------|----
               -2        -1           0           1         2
          Hydrophobic                                  Hydrophilic
```

**Fig 1E**

Fig. 2

```
MSFVGENSGVKMGSEDWEKDEPQCCLEDPAGSPLEPGPSLPTMNFVHTSPKVKNLNPKKFSIHDQD
MSFVGENSGVKMGSEDWEKDEPQCCLE

HKVLVLDSGNLIAVPDKNYIRPEIFFALASSLSSASAEKGSPILLGVSKGEFCLYCDKDKGQSHPS
                      EIFFALASSLSSASAEKGSPILLGVSKGEFCLYCDKDKGQSHPS

LQLKKEKLMKLAAQKESARRPFIFYRAQVGSWNMLESAAHPGWFICTSCNCNEPVGVTDKFENRKH
LQLKKEKLMKLAAQKESARRPFIFYRAQVGSWNMLESAAHPGWFICTSCNCNEPVGVTDKFENRKH

IEFSFQPVCKAEMSPSEVSD (SEQ ID NO:2)
IEFSFQPVCKAEMSPSEVSD (SEQ ID NO:9)
```

SEQUENCE LISTING

<110> ZymoGenetics, Inc.

<120> INTERLEUKIN-1 HOMOLOG ZIL1A4

<130> 98-59PC

<150> US 09/179.614
<151> 1998-10-27

<160> 44

<170> FastSEQ for Windows Version 3.0

<210> 1
<211> 1600
<212> DNA
<213> Homo sapiens

<220>
<221> CDS
<222> (847)...(1503)

<400> 1

```
ccgggcaggt ccagaagcta ttaccttaat tggttatgtg gatttcccct catactgagc     60
agctgtgtgt ggtgttgtaa aacatagcca tacacagtaa ctgacaaggg caaatgtgat    120
ggaaaaatgc aaggaagtgc agataaatag ctaatgggct gtagaaggaa gctagtcctt    180
ggagggcttg atcaaggaag gtccttttgc atgtcacctt tgaagaagag gggacataga    240
agaggtatag tgcatcccgg agtgtacctg gaagggaaca tgaaaagagg acatttttct    300
ctgggacatg gggactccac ttgcatgaac tctggaattg ggcaaagaa ccatcatgag     360
aacaagggct tccttgaacc tcccaggctc attggctgat ctaaaccctg tgtcccctct    420
ttccttcact ctcctctgtt ttctatacct gtattattgg actggactgg aagccacctg    480
atctatcaca agtaccttga aatgtgttga ataggtgtgg cacagtcctt agcagagtgg    540
cactaccccc acaggaattt gtttatacct ttggcatgga aaatagcagg aaatgagtga    600
tcactgataa ctgaggatgc tatttattat tggccaaagg aatacttgtg ttgtatttgc    660
ataaccactc acaaactgtt gattacaaat gagtaccaga cctagctcct tcaagtaaag    720
gatcctgaga actgaaggca aacagagctc caggagtcca agacagagcc acagaccacg    780
aggatccctg gcccaggtct tggacttcat tccattttct gttgagtaat aaactcaacg    840
```

```
ttgaaa atg tcc ttt gtg ggg gag aac tca gga gtg aaa atg ggc tct      888
       Met Ser Phe Val Gly Glu Asn Ser Gly Val Lys Met Gly Ser
        1               5                  10

gag gac tgg gaa aaa gat gaa ccc cag tgc tgc tta gaa gac ccg gct     936
Glu Asp Trp Glu Lys Asp Glu Pro Gln Cys Cys Leu Glu Asp Pro Ala
 15                  20                  25                  30

gga agc ccc ctg gaa cca ggc cca agc ctc ccc acc atg aat ttt gtt     984
Gly Ser Pro Leu Glu Pro Gly Pro Ser Leu Pro Thr Met Asn Phe Val
                35                  40                  45

cac aca agt cca aag gtg aag aac tta aac ccg aag aaa ttc agc att    1032
His Thr Ser Pro Lys Val Lys Asn Leu Asn Pro Lys Lys Phe Ser Ile
            50                  55                  60

cat gac cag gat cac aaa gta ctg gtc ctg gac tct ggg aat ctc ata    1080
His Asp Gln Asp His Lys Val Leu Val Leu Asp Ser Gly Asn Leu Ile
        65                  70                  75

gca gtt cca gat aaa aac tac ata cgc cca gag atc ttc ttt gca tta    1128
Ala Val Pro Asp Lys Asn Tyr Ile Arg Pro Glu Ile Phe Phe Ala Leu
    80                  85                  90

gcc tca tcc ttg agc tca gcc tct gcg gag aaa gga agt ccg att ctc    1176
Ala Ser Ser Leu Ser Ser Ala Ser Ala Glu Lys Gly Ser Pro Ile Leu
 95                 100                 105                 110

ctg ggg gtc tct aaa ggg gag ttt tgt ctc tac tgt gac aag gat aaa    1224
Leu Gly Val Ser Lys Gly Glu Phe Cys Leu Tyr Cys Asp Lys Asp Lys
                115                 120                 125

gga caa agt cat cca tcc ctt cag ctg aag aag gag aaa ctg atg aag    1272
Gly Gln Ser His Pro Ser Leu Gln Leu Lys Lys Glu Lys Leu Met Lys
            130                 135                 140

ctg gct gcc caa aag gaa tca gca cgc cgg ccc ttc atc ttt tat agg    1320
Leu Ala Ala Gln Lys Glu Ser Ala Arg Arg Pro Phe Ile Phe Tyr Arg
        145                 150                 155

gct cag gtg ggc tcc tgg aac atg ctg gag tcg gcg gct cac ccc gga    1368
Ala Gln Val Gly Ser Trp Asn Met Leu Glu Ser Ala Ala His Pro Gly
    160                 165                 170
```

```
tgg ttc atc tgc acc tcc tgc aat tgt aat gag cct gtt ggg gtg aca      1416
Trp Phe Ile Cys Thr Ser Cys Asn Cys Asn Glu Pro Val Gly Val Thr
175                 180                 185                 190

gat aaa ttt gag aac agg aaa cac att gaa ttt tca ttt caa cca gtt      1464
Asp Lys Phe Glu Asn Arg Lys His Ile Glu Phe Ser Phe Gln Pro Val
                195                 200                 205

tgc aaa gct gaa atg agc ccc agt gag gtc agc gat tag gaaactgccc       1513
Cys Lys Ala Glu Met Ser Pro Ser Glu Val Ser Asp  *
            210                 215

cattgaacgc cttcctcgct aatttgaact aattgtataa aaacaccaaa cctgctcact    1573
aaaaaaaaaa aaaaaaaaaa aaaaaaa                                         1600
```

<210> 2
<211> 218
<212> PRT
<213> Homo sapiens

<400> 2

```
Met Ser Phe Val Gly Glu Asn Ser Gly Val Lys Met Gly Ser Glu Asp
 1               5                  10                  15
Trp Glu Lys Asp Glu Pro Gln Cys Cys Leu Glu Asp Pro Ala Gly Ser
            20                  25                  30
Pro Leu Glu Pro Gly Pro Ser Leu Pro Thr Met Asn Phe Val His Thr
        35                  40                  45
Ser Pro Lys Val Lys Asn Leu Asn Pro Lys Lys Phe Ser Ile His Asp
    50                  55                  60
Gln Asp His Lys Val Leu Val Leu Asp Ser Gly Asn Leu Ile Ala Val
65                  70                  75                  80
Pro Asp Lys Asn Tyr Ile Arg Pro Glu Ile Phe Phe Ala Leu Ala Ser
                85                  90                  95
Ser Leu Ser Ser Ala Ser Ala Glu Lys Gly Ser Pro Ile Leu Leu Gly
            100                 105                 110
Val Ser Lys Gly Glu Phe Cys Leu Tyr Cys Asp Lys Asp Lys Gly Gln
        115                 120                 125
Ser His Pro Ser Leu Gln Leu Lys Lys Glu Lys Leu Met Lys Leu Ala
    130                 135                 140
Ala Gln Lys Glu Ser Ala Arg Arg Pro Phe Ile Phe Tyr Arg Ala Gln
145                 150                 155                 160
Val Gly Ser Trp Asn Met Leu Glu Ser Ala Ala His Pro Gly Trp Phe
                165                 170                 175
Ile Cys Thr Ser Cys Asn Cys Asn Glu Pro Val Gly Val Thr Asp Lys
            180                 185                 190
```

Phe Glu Asn Arg Lys His Ile Glu Phe Ser Phe Gln Pro Val Cys Lys
195 200 205
Ala Glu Met Ser Pro Ser Glu Val Ser Asp
210 215

<210> 3
<211> 5
<212> PRT
<213> Artificial Sequence

<220>
<223> peptide motif

<221> VARIANT
<222> (1)...(1)
<223> Xaa is Leu or Phe

<221> VARIANT
<222> (2)...(2)
<223> Xaa is Glu or Thr

<221> VARIANT
<222> (4)...(4)
<223> Xaa is Ala or Val

<221> VARIANT
<222> (5)...(5)
<223> Xaa is Ala or Gln

<400> 3

Xaa Xaa Ser Xaa Xaa
1 5

<210> 4
<211> 5
<212> PRT
<213> Artificial Sequence

<220>
<223> peptide motif

<221> VARIANT
<222> (2)...(2)
<223> Xaa is Gly or Asn
<221> VARIANT
<222> (3)...(3)
<223> Xaa is Leu or Trp

<221> VARIANT
<222> (4)...(4)
<223> Xaa is Phe or Tyr

<221> VARIANT
<222> (5)...(5)
<223> Xaa is Ile or Leu

<400> 4

Pro Xaa Xaa Xaa Xaa
1 5

<210> 5
<211> 5
<212> PRT
<213> Artificial Sequence

<220>
<223> peptide motif

<221> VARIANT
<222> (1)...(1)
<223> Xaa is Pro or Trp

<221> VARIANT
<222> (2)...(2)
<223> Xaa is Val or Leu

<221> VARIANT
<222> (3)...(3)
<223> Xaa is Ser, Cys, Phe, Arg, Ile or Gly

<221> VARIANT
<222> (4)...(4)
<223> Xaa is Leu or Val

<221> VARIANT
<222> (5)...(5)
<223> Xaa is Thr, Ala or Gly

<400> 5

Xaa Xaa Xaa Xaa Xaa
1 5

<210> 6
<211> 6
<212> PRT
<213> Artificial Sequence

<220>
<223> peptide motif

<221> VARIANT
<222> (1)...(1)
<223> Xaa is Thr or Ile

<221> VARIANT
<222> (2)...(2)
<223> Xaa is Asp, Lys or Glu

<221> VARIANT
<222> (4)...(4)
<223> Xaa is Ser, Tyr, Thr or Gln

<221> VARIANT
<222> (5)...(5)
<223> Xaa is Phe, Met or Ile

<221> VARIANT
<222> (6)...(6)
<223> Xaa is Gln, Asn, Ile or Leu

<400> 6

```
Xaa Xaa Phe Xaa Xaa Xaa
1               5
```

<210> 7
<211> 218
<212> PRT
<213> Artificial Sequence

<220>
<223> variant protein

<221> VARIANT
<222> (90)...(90)
<223> Xaa is Lys, Glu, or Ile

<221> VARIANT
<222> (155)...(155)
<223> Xaa is Ala, Ile, or Thr

<221> VARIANT
<222> (167)...(167)
<223> Xaa is Leu or Phe

<221> VARIANT
<222> (168)...(168)
<223> Xaa is Glu or Thr

<221> VARIANT
<222> (170)...(170)
<223> Xaa is Ala or Val

<221> VARIANT
<222> (171)...(171)
<223> Xaa is Ala or Gln

<221> VARIANT
<222> (174)...(174)
<223> Xaa is Gly or Asn

<221> VARIANT
<222> (175)...(175)
<223> Xaa is Leu or Trp

<221> VARIANT
<222> (176)...(176)
<223> Xaa is Phe or Tyr

<221> VARIANT
<222> (177)...(177)
<223> Xaa is Ile or Leu

<221> VARIANT
<222> (186)...(186)
<223> Xaa is Pro or Trp

<221> VARIANT
<222> (187)...(187)
<223> Xaa is Val or Leu

<221> VARIANT
<222> (188)...(188)
<223> Xaa is Ser. Cys, Phe, Arg, Ile, or Gly

<221> VARIANT
<222> (189)...(189)
<223> Xaa is Leu or Val

<221> VARIANT
<222> (190)...(190)
<223> Xaa is Thr, Ala, or Gly

<221> VARIANT
<222> (199)...(199)
<223> Xaa is Thr or Ile

<221> VARIANT
<222> (200)...(200)
<223> Xaa is Lys. Asp, or Glu

<221> VARIANT
<222> (202)...(202)
<223> Xaa is Ser. Tyr, Thr, or Gln

<221> VARIANT
<222> (203)...(203)
<223> Xaa is Phe, Met, or Ile

<221> VARIANT
<222> (204)...(204)
<223> Xaa is Gln, Asn, Ile, or Leu

<400> 7

```
Met Ser Phe Val Gly Glu Asn Ser Gly Val Lys Met Gly Ser Glu Asp
1               5                   10                  15
Trp Glu Lys Asp Glu Pro Gln Cys Cys Leu Glu Asp Pro Ala Gly Ser
            20                  25                  30
Pro Leu Glu Pro Gly Pro Ser Leu Pro Thr Met Asn Phe Val His Thr
        35                  40                  45
Ser Pro Lys Val Lys Asn Leu Asn Pro Lys Lys Phe Ser Ile His Asp
    50                  55                  60
Gln Asp His Lys Val Leu Val Leu Asp Ser Gly Asn Leu Ile Ala Val
65                  70                  75                  80

Pro Asp Lys Asn Tyr Ile Arg Pro Glu Xaa Phe Phe Ala Leu Ala Ser
                85                  90                  95
Ser Leu Ser Ser Ala Ser Ala Glu Lys Gly Ser Pro Ile Leu Leu Gly
            100                 105                 110
Val Ser Lys Gly Glu Phe Cys Leu Tyr Cys Asp Lys Asp Lys Gly Gln
        115                 120                 125
Ser His Pro Ser Leu Gln Leu Lys Lys Glu Lys Leu Met Lys Leu Ala
    130                 135                 140
Ala Gln Lys Glu Ser Ala Arg Arg Pro Phe Xaa Phe Tyr Arg Ala Gln
145                 150                 155                 160
Val Gly Ser Trp Asn Met Xaa Xaa Ser Xaa Xaa His Pro Xaa Xaa Xaa
                165                 170                 175
Xaa Cys Thr Ser Cys Asn Cys Asn Glu Xaa Xaa Xaa Xaa Xaa Asp Lys
            180                 185                 190
Phe Glu Asn Arg Lys His Xaa Xaa Phe Xaa Xaa Xaa Pro Val Cys Lys
        195                 200                 205
Ala Glu Met Ser Pro Ser Glu Val Ser Asp
    210                 215
```

<210> 8
<211> 636
<212> DNA
<213> Homo sapiens

<220>
<221> CDS
<222> (135)...(608)

<400> 8

```
gaattcggct tcctgagaac tgaaggcaaa cagagctcca ggagtccaag acagagccac     60
agaccacgag gatccctggc ccaggtcttg gacttcattc cattttctgt tgagtaataa    120
actcaacgtt gaaa atg tcc ttt gtg ggg gag aac tca gga gtg aaa atg      170
                Met Ser Phe Val Gly Glu Asn Ser Gly Val Lys Met
                1               5                   10

ggc tct gag gac tgg gaa aaa gat gaa ccc cag tgc tgc tta gaa gag      218
Gly Ser Glu Asp Trp Glu Lys Asp Glu Pro Gln Cys Cys Leu Glu Glu
        15                  20                  25

atc ttc ttt gca tta gcc tca tcc ttg agc tca gcc tct gcg gag aaa      266
Ile Phe Phe Ala Leu Ala Ser Ser Leu Ser Ser Ala Ser Ala Glu Lys
    30                  35                  40

gga agt ccg att ctc ctg ggg gtc tct aaa ggg gag ttt tgt ctc tac      314
Gly Ser Pro Ile Leu Leu Gly Val Ser Lys Gly Glu Phe Cys Leu Tyr
45                  50                  55                  60

tgt gac aag gat aaa gga caa agt cat cca tcc ctt cag ctg aag aag      362
Cys Asp Lys Asp Lys Gly Gln Ser His Pro Ser Leu Gln Leu Lys Lys
                65                  70                  75

gag aaa ctg atg aag ctg gct gcc caa aag gaa tca gca cgc cgg ccc      410
Glu Lys Leu Met Lys Leu Ala Ala Gln Lys Glu Ser Ala Arg Arg Pro
            80                  85                  90

ttc atc ttt tat agg gct cag gtg ggc tcc tgg aac atg ctg gag tcg      458
Phe Ile Phe Tyr Arg Ala Gln Val Gly Ser Trp Asn Met Leu Glu Ser
        95                  100                 105

gcg gct cac ccc gga tgg ttc atc tgc acc tcc tgc aat tgt aat gag      506
Ala Ala His Pro Gly Trp Phe Ile Cys Thr Ser Cys Asn Cys Asn Glu
    110                 115                 120

cct gtt ggg gtg aca gat aaa ttt gag aac agg aaa cac att gaa ttt      554
Pro Val Gly Val Thr Asp Lys Phe Glu Asn Arg Lys His Ile Glu Phe
125                 130                 135                 140

tca ttt caa cca gtt tgc aaa gct gaa atg agc ccc agt gag gtc agc      602
Ser Phe Gln Pro Val Cys Lys Ala Glu Met Ser Pro Ser Glu Val Ser
                145                 150                 155

gat tag gaaactgccc cattgaaaag ccgaattc                               636
Asp  *
```

<210> 9
<211> 157

<212> PRT
<213> Homo sapiens

<400> 9

```
Met Ser Phe Val Gly Glu Asn Ser Gly Val Lys Met Gly Ser Glu Asp
 1               5                  10                  15
Trp Glu Lys Asp Glu Pro Gln Cys Cys Leu Glu Glu Ile Phe Phe Ala
            20                  25                  30
Leu Ala Ser Ser Leu Ser Ser Ala Ser Ala Glu Lys Gly Ser Pro Ile
        35                  40                  45

Leu Leu Gly Val Ser Lys Gly Glu Phe Cys Leu Tyr Cys Asp Lys Asp
    50                  55                  60
Lys Gly Gln Ser His Pro Ser Leu Gln Leu Lys Lys Glu Lys Leu Met
65                  70                  75                  80
Lys Leu Ala Ala Gln Lys Glu Ser Ala Arg Arg Pro Phe Ile Phe Tyr
                85                  90                  95
Arg Ala Gln Val Gly Ser Trp Asn Met Leu Glu Ser Ala Ala His Pro
            100                 105                 110
Gly Trp Phe Ile Cys Thr Ser Cys Asn Cys Asn Glu Pro Val Gly Val
        115                 120                 125
Thr Asp Lys Phe Glu Asn Arg Lys His Ile Glu Phe Ser Phe Gln Pro
    130                 135                 140
Val Cys Lys Ala Glu Met Ser Pro Ser Glu Val Ser Asp
145                 150                 155
```

<210> 10
<211> 157
<212> PRT
<213> Artificial Sequence

<220>
<223> variant protein

<221> VARIANT
<222> (29)...(29)
<223> Xaa is Lys, Glu, or Ile

<221> VARIANT
<222> (94)...(94)
<223> Xaa is Ala, Ile, or Thr

<221> VARIANT
<222> (106)...(106)
<223> Xaa is Leu or Phe

<221> VARIANT
<222> (107)...(107)

<223> Xaa is Glu or Thr

<221> VARIANT
<222> (109)...(109)
<223> Xaa is Ala or Val

<221> VARIANT
<222> (110)...(110)
<223> Xaa is Ala or Gln

<221> VARIANT
<222> (113)...(113)
<223> Xaa is Gly or Asn

<221> VARIANT
<222> (114)...(114)
<223> Xaa is Leu or Trp

<221> VARIANT
<222> (115)...(115)
<223> Xaa is Phe or Tyr

<221> VARIANT
<222> (116)...(116)
<223> Xaa is Ile or Leu

<221> VARIANT
<222> (125)...(125)
<223> Xaa is Pro or Trp

<221> VARIANT
<222> (126)...(126)
<223> Xaa is Val or Leu

<221> VARIANT
<222> (127)...(127)
<223> Xaa is Ser, Cys, Phe, Arg. Ile, or Gly

<221> VARIANT
<222> (128)...(128)
<223> Xaa is Leu or Val

<221> VARIANT
<222> (129)...(129)
<223> Xaa is Thr, Ala, or Gly

<221> VARIANT
<222> (138)...(138)
<223> Xaa is Thr or Ile

<221> VARIANT
<222> (139)...(139)
<223> Xaa is Lys, Asp, or Glu

<221> VARIANT
<222> (141)...(141)
<223> Xaa is Ser. Tyr, Thr, or Gln

<221> VARIANT
<222> (142)...(142)
<223> Xaa is Phe, Met, or Ile

<221> VARIANT
<222> (143)...(143)
<223> Xaa is Gln, Asn, Ile, or Leu

<400> 10

```
Met Ser Phe Val Gly Glu Asn Ser Gly Val Lys Met Gly Ser Glu Asp
 1               5                  10                  15
Trp Glu Lys Asp Glu Pro Gln Cys Cys Leu Glu Glu Xaa Phe Phe Ala
            20                  25                  30
Leu Ala Ser Ser Leu Ser Ser Ala Ser Ala Glu Lys Gly Ser Pro Ile
        35                  40                  45
Leu Leu Gly Val Ser Lys Gly Glu Phe Cys Leu Tyr Cys Asp Lys Asp
    50                  55                  60
Lys Gly Gln Ser His Pro Ser Leu Gln Leu Lys Lys Glu Lys Leu Met
65                  70                  75                  80
Lys Leu Ala Ala Gln Lys Glu Ser Ala Arg Arg Pro Phe Xaa Phe Tyr
                85                  90                  95
Arg Ala Gln Val Gly Ser Trp Asn Met Xaa Xaa Ser Xaa Xaa His Pro
            100                 105                 110
Xaa Xaa Xaa Xaa Cys Thr Ser Cys Asn Cys Asn Glu Xaa Xaa Xaa Xaa
        115                 120                 125
Xaa Asp Lys Phe Glu Asn Arg Lys His Xaa Xaa Phe Xaa Xaa Xaa Pro
    130                 135                 140
Val Cys Lys Ala Glu Met Ser Pro Ser Glu Val Ser Asp
145                 150                 155
```

<210> 11
<211> 218
<212> PRT
<213> Artificial Sequence

<220>
<223> variant protein

<221> VARIANT
<222> (90)...(90)
<223> Xaa is Lys. Glu or Ile

<221> VARIANT
<222> (155)...(155)
<223> Xaa is Ala, Ile or Thr

<221> VARIANT
<222> (200)...(200)
<223> Xaa is Lys, Asp or Glu

<400> 11

```
Met Ser Phe Val Gly Glu Asn Ser Gly Val Lys Met Gly Ser Glu Asp
 1               5                  10                  15
Trp Glu Lys Asp Glu Pro Gln Cys Cys Leu Glu Asp Pro Ala Gly Ser
            20                  25                  30
Pro Leu Glu Pro Gly Pro Ser Leu Pro Thr Met Asn Phe Val His Thr
        35                  40                  45
Ser Pro Lys Val Lys Asn Leu Asn Pro Lys Lys Phe Ser Ile His Asp
    50                  55                  60
Gln Asp His Lys Val Leu Val Leu Asp Ser Gly Asn Leu Ile Ala Val
65                  70                  75                  80
Pro Asp Lys Asn Tyr Ile Arg Pro Glu Xaa Phe Phe Ala Leu Ala Ser
                85                  90                  95
Ser Leu Ser Ser Ala Ser Ala Glu Lys Gly Ser Pro Ile Leu Leu Gly
            100                 105                 110
Val Ser Lys Gly Glu Phe Cys Leu Tyr Cys Asp Lys Asp Lys Gly Gln
        115                 120                 125
Ser His Pro Ser Leu Gln Leu Lys Lys Glu Lys Leu Met Lys Leu Ala
    130                 135                 140
Ala Gln Lys Glu Ser Ala Arg Arg Pro Phe Xaa Phe Tyr Arg Ala Gln
145                 150                 155                 160
Val Gly Ser Trp Asn Met Leu Glu Ser Ala Ala His Pro Gly Trp Phe
                165                 170                 175
Ile Cys Thr Ser Cys Asn Cys Asn Glu Pro Val Gly Val Thr Asp Lys
            180                 185                 190
Phe Glu Asn Arg Lys His Ile Xaa Phe Ser Phe Gln Pro Val Cys Lys
        195                 200                 205
Ala Glu Met Ser Pro Ser Glu Val Ser Asp
    210                 215
```

<210> 12
<211> 157
<212> PRT
<213> Artificial Sequence

<220>
<223> variant protein

<221> VARIANT
<222> (29)...(29)
<223> Xaa is Lys, Glu, or Ile

<221> VARIANT
<222> (94)...(94)
<223> Xaa is Ala, Ile, or Thr

<221> VARIANT
<222> (139)...(139)
<223> Xaa is Lys, Asp, or Glu

<400> 12

Met Ser Phe Val Gly Glu Asn Ser Gly Val Lys Met Gly Ser Glu Asp
1 5 10 15
Trp Glu Lys Asp Glu Pro Gln Cys Cys Leu Glu Glu Xaa Phe Phe Ala
20 25 30
Leu Ala Ser Ser Leu Ser Ser Ala Ser Ala Glu Lys Gly Ser Pro Ile
35 40 45
Leu Leu Gly Val Ser Lys Gly Glu Phe Cys Leu Tyr Cys Asp Lys Asp
50 55 60
Lys Gly Gln Ser His Pro Ser Leu Gln Leu Lys Lys Glu Lys Leu Met
65 70 75 80
Lys Leu Ala Ala Gln Lys Glu Ser Ala Arg Arg Pro Phe Xaa Phe Tyr
85 90 95
Arg Ala Gln Val Gly Ser Trp Asn Met Leu Glu Ser Ala Ala His Pro
100 105 110
Gly Trp Phe Ile Cys Thr Ser Cys Asn Cys Asn Glu Pro Val Gly Val
115 120 125
Thr Asp Lys Phe Glu Asn Arg Lys His Ile Xaa Phe Ser Phe Gln Pro
130 135 140
Val Cys Lys Ala Glu Met Ser Pro Ser Glu Val Ser Asp
145 150 155

<210> 13
<211> 657
<212> DNA
<213> Artificial Sequence

<220>
<221> CDS
<222> (1)...(657)

<221> variation
<222> (598)...(601)
<223> degenerate codon #200 (aar) encodes Lys

<223> variant DNA

<400> 13

```
atg tcc ttt gtg ggg gag aac tca gga gtg aaa atg ggc tct gag gac      48
Met Ser Phe Val Gly Glu Asn Ser Gly Val Lys Met Gly Ser Glu Asp
 1               5                  10                  15

tgg gaa aaa gat gaa ccc cag tgc tgc tta gaa gac ccg gct gga agc      96
Trp Glu Lys Asp Glu Pro Gln Cys Cys Leu Glu Asp Pro Ala Gly Ser
            20                  25                  30

ccc ctg gaa cca ggc cca agc ctc ccc acc atg aat ttt gtt cac aca     144
Pro Leu Glu Pro Gly Pro Ser Leu Pro Thr Met Asn Phe Val His Thr
        35                  40                  45

agt cca aag gtg aag aac tta aac ccg aag aaa ttc agc att cat gac     192
Ser Pro Lys Val Lys Asn Leu Asn Pro Lys Lys Phe Ser Ile His Asp
    50                  55                  60

cag gat cac aaa gta ctg gtc ctg gac tct ggg aat ctc ata gca gtt     240
Gln Asp His Lys Val Leu Val Leu Asp Ser Gly Asn Leu Ile Ala Val
65                  70                  75                  80

cca gat aaa aac tac ata cgc cca gag atc ttc ttt gca tta gcc tca     288
Pro Asp Lys Asn Tyr Ile Arg Pro Glu Ile Phe Phe Ala Leu Ala Ser
                85                  90                  95

tcc ttg agc tca gcc tct gcg gag aaa gga agt ccg att ctc ctg ggg     336
Ser Leu Ser Ser Ala Ser Ala Glu Lys Gly Ser Pro Ile Leu Leu Gly
            100                 105                 110

gtc tct aaa ggg gag ttt tgt ctc tac tgt gac aag gat aaa gga caa     384
Val Ser Lys Gly Glu Phe Cys Leu Tyr Cys Asp Lys Asp Lys Gly Gln
        115                 120                 125

agt cat cca tcc ctt cag ctg aag aag gag aaa ctg atg aag ctg gct     432
Ser His Pro Ser Leu Gln Leu Lys Lys Glu Lys Leu Met Lys Leu Ala
    130                 135                 140
```

```
gcc caa aag gaa tca gca cgc cgg ccc ttc atc ttt tat agg gct cag        480
Ala Gln Lys Glu Ser Ala Arg Arg Pro Phe Ile Phe Tyr Arg Ala Gln
145                 150                 155                 160

gtg ggc tcc tgg aac atg ctg gag tcg gcg gct cac ccc gga tgg ttc        528
Val Gly Ser Trp Asn Met Leu Glu Ser Ala Ala His Pro Gly Trp Phe
                165                 170                 175

atc tgc acc tcc tgc aat tgt aat gag cct gtt ggg gtg aca gat aaa        576
Ile Cys Thr Ser Cys Asn Cys Asn Glu Pro Val Gly Val Thr Asp Lys
            180                 185                 190

ttt gag aac agg aaa cac att aar ttt tca ttt caa cca gtt tgc aaa        624
Phe Glu Asn Arg Lys His Ile Xaa Phe Ser Phe Gln Pro Val Cys Lys
        195                 200                 205

gct gaa atg agc ccc agt gag gtc agc gat tag                            657
Ala Glu Met Ser Pro Ser Glu Val Ser Asp  *
    210                 215
```

<210> 14
<211> 218
<212> PRT
<213> Artificial Sequence

<220>
<223> variant protein

<400> 14

```
Met Ser Phe Val Gly Glu Asn Ser Gly Val Lys Met Gly Ser Glu Asp
1               5                   10                  15
Trp Glu Lys Asp Glu Pro Gln Cys Cys Leu Glu Asp Pro Ala Gly Ser
            20                  25                  30
Pro Leu Glu Pro Gly Pro Ser Leu Pro Thr Met Asn Phe Val His Thr
        35                  40                  45
Ser Pro Lys Val Lys Asn Leu Asn Pro Lys Lys Phe Ser Ile His Asp
    50                  55                  60
Gln Asp His Lys Val Leu Val Leu Asp Ser Gly Asn Leu Ile Ala Val
65                  70                  75                  80
Pro Asp Lys Asn Tyr Ile Arg Pro Glu Ile Phe Phe Ala Leu Ala Ser
                85                  90                  95
Ser Leu Ser Ser Ala Ser Ala Glu Lys Gly Ser Pro Ile Leu Leu Gly
            100                 105                 110
```

Val Ser Lys Gly Glu Phe Cys Leu Tyr Cys Asp Lys Asp Lys Gly Gln
115 120 125
Ser His Pro Ser Leu Gln Leu Lys Lys Glu Lys Leu Met Lys Leu Ala
130 135 140
Ala Gln Lys Glu Ser Ala Arg Arg Pro Phe Ile Phe Tyr Arg Ala Gln
145 150 155 160
Val Gly Ser Trp Asn Met Leu Glu Ser Ala Ala His Pro Gly Trp Phe
165 170 175
Ile Cys Thr Ser Cys Asn Cys Asn Glu Pro Val Gly Val Thr Asp Lys
180 185 190
Phe Glu Asn Arg Lys His Ile Lys Phe Ser Phe Gln Pro Val Cys Lys
195 200 205
Ala Glu Met Ser Pro Ser Glu Val Ser Asp
210 215

<210> 15
<211> 157
<212> PRT
<213> Artificial Sequence

<220>
<223> variant protein

<400> 15

Met Ser Phe Val Gly Glu Asn Ser Gly Val Lys Met Gly Ser Glu Asp
1 5 10 15
Trp Glu Lys Asp Glu Pro Gln Cys Cys Leu Glu Glu Ile Phe Phe Ala
20 25 30
Leu Ala Ser Ser Leu Ser Ser Ala Ser Ala Glu Lys Gly Ser Pro Ile
35 40 45
Leu Leu Gly Val Ser Lys Gly Glu Phe Cys Leu Tyr Cys Asp Lys Asp
50 55 60
Lys Gly Gln Ser His Pro Ser Leu Gln Leu Lys Lys Glu Lys Leu Met
65 70 75 80
Lys Leu Ala Ala Gln Lys Glu Ser Ala Arg Arg Pro Phe Ile Phe Tyr
85 90 95
Arg Ala Gln Val Gly Ser Trp Asn Met Leu Glu Ser Ala Ala His Pro
100 105 110
Gly Trp Phe Ile Cys Thr Ser Cys Asn Cys Asn Glu Pro Val Gly Val
115 120 125
Thr Asp Lys Phe Glu Asn Arg Lys His Ile Lys Phe Ser Phe Gln Pro
130 135 140
Val Cys Lys Ala Glu Met Ser Pro Ser Glu Val Ser Asp
145 150 155

<210> 16

<211> 654
<212> DNA
<213> Artificial Sequence

<220>
<221> misc_feature
<222> (1)...(654)
<223> n is any nucleotide

<223> degenerate sequence

<400> 16

```
atgwsnttyg tnggngaraa ywsnggngtn aaratgggnw sngargaytg ggaraargay     60
garccncart gytgyytnga rgayccngcn ggnwsnccny tngarccngg nccnwsnytn    120
ccnacnatga ayttygtnca yacnwsnccn aargtnaara ayytnaaycc naaraartty    180
wsnathcayg aycargayca yaargtnytn gtnytngayw snggnaayyt nathgcngtn    240
ccngayaara aytayathmg nccngarath ttyttygcny tngcnwsnws nytnwsnwsn    300
gcnwsngcng araarggnws nccnathytn ytnggngtnw snaarggnga rttytgyytn    360
taytgygaya argayaargg ncarwsncay ccnwsnytnc arytnaaraa rgaraarytn    420
atgaarytng cngcncaraa rgarwsngcn mgnmgnccnt tyathttyta ymgngcncar    480
gtnggnwsnt ggaayatgyt ngarwsngcn gcncayccng gntggttyat htgyacnwsn    540
tgyaaytgya aygarccngt nggngtnacn gayaarttyg araaymgnaa rcayathgar    600
ttywsnttyc arccngtntg yaargcngar atgwsnccnw sngargtnws ngay          654
```

<210> 17
<211> 654
<212> DNA
<213> Artificial Sequence

<220>
<221> variation
<222> (1)...(654)
<223> n is any nucleotide

<223> degenerate sequence

<400> 17

```
atgwsnttyg tnggngaraa ywsnggngtn aaratgggnw sngargaytg ggaraargay     60
garccncart gytgyytnga rgayccngcn ggnwsnccny tngarccngg nccnwsnytn    120
ccnacnatga ayttygtnca yacnwsnccn aargtnaara ayytnaaycc naaraartty    180
wsnathcayg aycargayca yaargtnytn gtnytngayw snggnaayyt nathgcngtn    240
ccngayaara aytayathmg nccngarath ttyttygcny tngcnwsnws nytnwsnwsn    300
gcnwsngcng araarggnws nccnathytn ytnggngtnw snaarggnga rttytgyytn    360
taytgygaya argayaargg ncarwsncay ccnwsnytnc arytnaaraa rgaraarytn    420
```

atgaarytng cngcncaraa rgarwsngcn mgnmgnccnt tyathttyta ymgngcncar 480
gtnggnwsnt ggaayatgyt ngarwsngcn gcncayccng gntggttyat htgyacnwsn 540
tgyaaytgya aygarccngt nggngtnacn gayaarttyg araaymgnaa rcayathaar 600
ttywsnttyc arccngtntg yaargcngar atgwsnccnw sngargtnws ngay 654

<210> 18
<211> 471
<212> DNA
<213> Artificial Sequence

<220>
<223> degenerate sequence

<221> misc_feature
<222> (1)...(471)
<223> n = A,T,C or G

<400> 18

atgwsnttyg tnggngaraa ywsnggngtn aaratgggnw sngargaytg ggaraargay 60
garccncart gytgyytnga rgarathtty ttygcnytng cnwsnwsnyt nwsnwsngcn 120
wsngcngara arggnwsncc nathytnytn ggngtnwsna arggngartt ytgyytntay 180
tgygayaarg ayaarggnca rwsncayccn wsnytncary tnaaraarga raarytnatg 240
aarytngcng cncaraarga rwsngcnmgn mgnccnttya thttytaymg ngcncargtn 300
ggnwsntgga ayatgytnga rwsngcngcn cayccnggnt ggttyathtg yacnwsntgy 360
aaytgyaayg arccngtngg ngtnacngay aarttygara aymgnaarca yathgartty 420
wsnttycarc cngtntgyaa rgcngaratg wsnccnwsng argtnwsnga y 471

<210> 19
<211> 471
<212> DNA
<213> Artificial Sequence

<220>
<223> degenerate sequence

<221> mise_feature
<222> (1)...(471)
<223> n = A,T,C or G

<400> 19

atgwsnttyg tnggngaraa ywsnggngtn aaratgggnw sngargaytg ggaraargay 60
garccncart gytgyytnga rgarathtty ttygcnytng cnwsnwsnyt nwsnwsngcn 120
wsngcngara arggnwsncc nathytnytn ggngtnwsna arggngartt ytgyytntay 180
tgygayaarg ayaarggnca rwsncayccn wsnytncary tnaaraarga raarytnatg 240
aarytngcng cncaraarga rwsngcnmgn mgnccnttya thttytaymg ngcncargtn 300

ggnwsntgga ayatgytnga rwsngcngcn cayccnggnt ggttyathtg yacnwsntgy 360
aaytgyaayg arccngtngg ngtnacngay aarttygara aymgnaarca yathaartty 420
wsnttycarc cngtntgyaa rgcngaratg wsnccnwsng argtnwsnga y 471

<210> 20
<211> 17
<212> DNA
<213> Artificial Sequence

<220>
<221> variation
<222> (1)...(17)
<223> n is any nucleotide

<223> oligonucleotide primer

<400> 20

cayccnggnt ggttyat . 17

<210> 21
<211> 17
<212> DNA
<213> Artificial Sequence

<220>
<221> variation
<222> (1)...(17)
<223> n is any nucleotide

<223> oligonucleotide primer

<400> 21

ydyccnrrny kntwyht 17

<210> 22
<211> 17
<212> DNA
<213> Artificial Sequence

<220>
<221> variation
<222> (1)...(17)
<223> n is any nucleotide

<223> oligonucleotide primer

<400> 22

adrwanmrny ynggrhr 17

<210> 23
<211> 17
<212> DNA
<213> Artificial Sequence

<220>
<221> variation
<222> (1)...(17)
<223> n is any nucleotide

<223> oligonucleotide primer

<400> 23

athgarttyw snttyca 17

<210> 24
<211> 17
<212> DNA
<213> Artificial Sequence

<220>
<221> variation
<222> (1)...(17)
<223> n is any nucleotide

<223> oligonucleotide primer

<400> 24

aynranttyh vnwtnyw 17

<210> 25
<211> 17
<212> DNA
<213> Artificial Sequence

<220>
<221> variation
<222> (1)...(17)
<223> n is any nucleotide

<223> oligonucleotide primer

<400> 25

wrnawnbdra antynrt 17

<210> 26
<211> 17
<212> DNA
<213> Artificial Sequence

<220>
<221> variation
<222> (1)...(17)
<223> n is any nucleotide

<223> oligonucleotide primer

<400> 26

aarttywsna thcayga 17

<210> 27
<211> 17
<212> DNA
<213> Artificial Sequence

<220>
<221> variation
<222> (1)...(17)
<223> n is any nucleotide

<223> oligonucleotide primer

<400> 27

rmntkyhbnh tnhrnga 17

<210> 28
<211> 17
<212> DNA
<213> Artificial Sequence

<220>
<221> variation
<222> (1)...(17)
<223> n is any nucleotide

<223> oligonucleotide primer

<400> 28

tcnydnadnv drmanky 17

<210> 29
<211> 17
<212> DNA
<213> Artificial Sequence

<220>
<221> variation
<222> (1)...(17)
<223> n is any nucleotide

<223> oligonucleotide primer

<400> 29

garttytgyy tntaytg 17

<210> 30
<211> 17
<212> DNA
<213> Artificial Sequence

<220>
<221> variation
<222> (1)...(17)
<223> n is any nucleotide

<223> oligonucleotide primer

<400> 30

vanhtntryb tnwvnks 17

<210> 31
<211> 17
<212> DNA
<213> Artificial Sequence

<220>
<221> variation
<222> (1)...(17)
<223> n is any nucleotide

<223> oligonucleotide primer

<400> 31

smnbwnavry anadntb 17

<210> 32
<211> 24
<212> DNA
<213> Artificial Sequence

<220>
<223> oligonucleotide primer

<400> 32

gacaaagtca tccatccctt cagc 24

<210> 33
<211> 22
<212> DNA
<213> Artificial Sequence

<220>
<223> oligonucleotide primer

<400> 33

cagcatgttc caggagccca cc 22

<210> 34
<211> 27
<212> DNA
<213> Artificial Sequence

<220>
<223> oligonucleotide primer

<400> 34

ccatcctaat acgactcact atagggc 27

<210> 35
<211> 22
<212> DNA
<213> Artificial Sequence

<220>
<223> oligonucleotide primer

<400> 35

cgccgactcc agcatgttcc ag 22

<210> 36
<211> 23
<212> DNA
<213> Artificial Sequence

<220>
<223> oligonucleotide primer

<400> 36

actcactata gggctcgagc cgc 23

<210> 37
<211> 24
<212> DNA
<213> Artificial Sequence

<220>
<223> oligonucleotide primer

<400> 37

gggcagccag cttcatcagt ttct 24

<210> 38
<213> 24
<212> DNA
<213> Artificial Sequence

<220>
<223> oligonucleotide primer

<400> 38

ggctcaggtg ggctcctgga acat 24

<210> 39
<211> 24
<212> DNA
<213> Artificial Sequence

<220>
<223> oligonucleotide primer

<400> 39

ggatggttca tctgcacctc ctgc 24

<210> 40
<211> 24
<212> DNA
<213> Artificial Sequence

<220>
<223> oligonucleotide primer

<400> 40

cctgagaact gaaggcaaac agag 24

<210> 41
<211> 24
<212> DNA
<213> Artificial Sequence

<220>
<223> oligonucleotide primer

<400> 41

ttcaatgggg cagtttccta atcg 24

<210> 42
<211> 6

<212> PRT
<213> Artificial Sequence

<220>
<223> peptide tag

<400> 42

Glu Tyr Met Pro Met Glu
1               5

<210> 43
<211> 18
<212> DNA
<213> Artificial Sequence

<220>
<223> oligonucleotide primer ZC20,360

<400> 43

tcccctcttt ccttcact 18

<210> 44
<211> 18
<212> DNA
<213> Artificial Sequence

<220>
<223> oligonucleotide primer ZC20,361

<400> 44

gtgccacacc tattcaac 18